(19) Europäisches Patentamt
European Patent Office
Office européen des brevets

(11) **EP 3 244 212 A1**

(12) **EUROPEAN PATENT APPLICATION**

(43) Date of publication:
**15.11.2017 Bulletin 2017/46**

(51) Int Cl.:
**G01N 33/68** (2006.01)     **A61K 39/35** (2006.01)
**C07K 14/415** (2006.01)

(21) Application number: **17000245.5**

(22) Date of filing: **15.02.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Euroimmun Medizinische Labordiagnostika AG**
**23560 Lübeck (DE)**

(72) Inventor: **The designation of the inventor has not yet been filed**

(54) **AN IMPROVED ASSAY FOR THE DIAGNOSIS OF PEANUT ALLERGY**

(57)     The present invention relates to a diagnostically useful carrier comprising a means for specifically capturing an antibody to Ara h 7 isotype 7.0201 in a sample from a subject; a method comprising the step detecting in a sample from a subject the presence or absence of an antibody to Ara h 7 isotype 7.0201 and a pharmaceutical composition comprising Ara h 7 isotype 7.0201 or a variant thereof.

**Fig. 1**

EP 3 244 212 A1

SEQUENCE LISTING

<110> EUROIMMUN Medizinische Labordiagnostika AG

<120> An improved assay for the diagnosis of peanut allergy

<130> 17PP016EP

<160> 18

<170> BiSSAP 1.3

<210> 1
<211> 139
<212> PRT
<213> Artificial Sequence

<220>
<223> SEQ ID NO1 (expressed sequence without signal sequence of Ara h 7.0101)

<400> 1
Thr Arg Trp Asp Pro Asp Arg Gly Ser Arg Gly Ser Arg Trp Asp Ala
1               5                   10                  15
Pro Ser Arg Gly Asp Asp Gln Cys Gln Arg Gln Leu Gln Arg Ala Asn
            20                  25                  30
Leu Arg Pro Cys Glu Glu His Met Arg Arg Arg Val Glu Gln Glu Gln
            35                  40                  45
Glu Gln Glu Gln Asp Glu Tyr Pro Tyr Ser Arg Arg Gly Ser Arg Gly
        50                  55                  60
Arg Gln Pro Gly Glu Ser Asp Glu Asn Gln Glu Gln Arg Cys Cys Asn
65                  70                  75                  80
Glu Leu Asn Arg Phe Gln Asn Asn Gln Arg Cys Met Cys Gln Ala Leu
                85                  90                  95
Gln Gln Ile Leu Gln Asn Gln Ser Phe Trp Val Pro Ala Gly Gln Glu
            100                 105                 110
Pro Val Ala Ser Asp Gly Glu Gly Ala Gln Glu Leu Ala Pro Glu Leu
            115                 120                 125
Arg Val Gln Val Thr Lys Pro Leu Arg Pro Leu
        130                 135

<210> 2
<211> 144
<212> PRT
<213> Artificial Sequence

<220>
<223> SEQ ID NO2 (expressed sequence without signal sequence of Ara h 7.0201)

<400> 2
Thr Arg Trp Asp Pro Asp Arg Gly Ser Arg Gly Ser Arg Trp Asp Ala
1               5                   10                  15
Pro Ser Arg Gly Asp Asp Gln Cys Gln Arg Gln Leu Gln Arg Ala Asn
            20                  25                  30
Leu Arg Pro Cys Glu Glu His Ile Arg Gln Arg Val Glu Lys Glu Gln
            35                  40                  45
Glu Gln Glu Gln Asp Glu Tyr Pro Tyr Ile Gln Arg Gly Ser Arg Gly
        50                  55                  60
Gln Arg Pro Gly Glu Ser Asp Glu Asp Gln Glu Gln Arg Cys Cys Asn
65                  70                  75                  80
Glu Leu Asn Arg Phe Gln Asn Asn Gln Arg Cys Met Cys Gln Ala Leu
                85                  90                  95

```
Gln Gln Ile Leu Gln Asn Gln Ser Phe Arg Phe Gln Gln Asp Arg Ser
            100                     105             110
Gln Leu His Gln Met Glu Arg Glu Leu Arg Asn Leu Pro Gln Asn Cys
            115                     120             125
Gly Phe Arg Ser Pro Ser Arg Cys Asp Leu Ser Ser Arg Thr Pro Tyr
            130             135                 140
```

<210> 3
<211> 138
<212> PRT
<213> Artificial Sequence

<220>
<223> SEQ ID NO3 (expressed sequence without signal sequence of Ara h
      7.0)

<400> 3
```
Thr Arg Trp Asp Pro Asp Arg Gly Ser Arg Gly Leu Arg Trp Asp Ala
1               5               10                  15
Pro Ser Arg Gly Asp Asp Gln Cys Gln Arg Gln Leu Gln Arg Ala Asn
            20                  25              30
Leu Arg Pro Cys Glu Glu His Ile Arg Gln Arg Val Glu Gln Glu Gln
            35                  40              45
Glu Gln Glu Gln Asp Glu Tyr Pro Tyr Ser Gln Arg Gly Ser Arg Gly
        50              55              60
Arg Arg Pro Gly Glu Ser Asp Glu Asp Gln Gln Arg Cys Cys Asn
65                  70                  75                  80
Glu Leu Asn Arg Phe Gln Asn Asn Gln Arg Cys Met Cys Gln Ala Leu
                85                  90                  95
Gln Gln Ile Leu Gln Asn Gln Ser Phe Arg Phe Gln Gln Asp Arg Ser
            100                     105             110
Gln Leu His Gln Asn Gly Glu Gly Ala Gln Glu Leu Ala Pro Glu Leu
            115                     120             125
Arg Val Gln Val Thr Lys Pro Leu Arg Pro
            130             135
```

<210> 4
<211> 151
<212> PRT
<213> Artificial Sequence

<220>
<223> SEQ ID NO4 (expressed sequence without signal sequence of Ara h
      2.0201)

<400> 4
```
Arg Gln Gln Trp Glu Leu Gln Gly Asp Arg Arg Cys Gln Ser Gln Leu
1               5               10                  15
Glu Arg Ala Asn Leu Arg Pro Cys Glu Gln His Leu Met Gln Lys Ile
            20                  25              30
Gln Arg Asp Glu Asp Ser Tyr Gly Arg Asp Pro Tyr Ser Pro Ser Gln
            35                  40              45
Asp Pro Tyr Ser Pro Ser Gln Asp Pro Asp Arg Arg Asp Pro Tyr Ser
        50              55              60
Pro Ser Pro Tyr Asp Arg Arg Gly Ala Gly Ser Ser Gln His Gln Glu
65                  70                  75                  80
Arg Cys Cys Asn Glu Leu Asn Glu Phe Glu Asn Asn Gln Arg Cys Met
                85                  90                  95
Cys Glu Ala Leu Gln Gln Ile Met Glu Asn Gln Ser Asp Arg Leu Gln
            100                     105             110
Gly Arg Gln Gln Glu Gln Gln Phe Lys Arg Glu Leu Arg Asn Leu Pro
```

```
              115                   120                   125
         Gln Gln Cys Gly Leu Arg Ala Pro Gln Arg Cys Asp Leu Glu Val Glu
              130                   135                   140
         Ser Gly Gly Arg Asp Arg Tyr
         145                   150


         <210> 5
         <211> 124
         <212> PRT
         <213> Artificial Sequence

         <220>
         <223> SEQ ID NO5 (expressed sequence without signal sequence of Ara h
               6.0101)

         <400> 5
         Met Arg Arg Glu Arg Gly Arg Gln Gly Asp Ser Ser Ser Cys Glu Arg
         1                   5                   10                  15
         Gln Val Asp Arg Val Asn Leu Lys Pro Cys Glu Gln His Ile Met Gln
                      20                  25                  30
         Arg Ile Met Gly Glu Gln Glu Gln Tyr Asp Ser Tyr Asp Ile Arg Ser
                      35                  40                  45
         Thr Arg Ser Ser Asp Gln Gln Gln Arg Cys Cys Asp Glu Leu Asn Glu
              50                  55                  60
         Met Glu Asn Thr Gln Arg Cys Met Cys Glu Ala Leu Gln Gln Ile Met
         65                  70                  75                  80
         Glu Asn Gln Cys Asp Arg Leu Gln Asp Arg Gln Met Val Gln Gln Phe
                          85                  90                  95
         Lys Arg Glu Leu Met Asn Leu Pro Gln Gln Cys Asn Phe Arg Ala Pro
                      100                 105                 110
         Gln Arg Cys Asp Leu Asp Val Ser Gly Gly Arg Cys
                      115                 120


         <210> 6
         <211> 30
         <212> PRT
         <213> Artificial Sequence

         <220>
         <223> SEQ ID NO6 (C-terminus of Ara h 7 isotype 7.0201)

         <400> 6
         His Gln Met Glu Arg Glu Leu Arg Asn Leu Pro Gln Asn Cys Gly Phe
         1                   5                   10                  15
         Arg Ser Pro Ser Arg Cys Asp Leu Ser Ser Arg Thr Pro Tyr
                      20                  25                  30


         <210> 7
         <211> 10
         <212> PRT
         <213> Artificial Sequence

         <220>
         <223> SEQ ID NO7 (C-terminus of Ara h 7 isotype 7.0201)

         <400> 7
         Asn Cys Gly Phe Arg Ser Pro Ser Arg Cys
         1                   5                   10


         <210> 8
         <211> 6
         <212> PRT
         <213> Artificial Sequence
```

<220>
<223> SEQ ID NO8 (reactive epitope from C-terminus of Ara h 7 isotype 7.0201)

<400> 8
Gly Phe Arg Ser Pro Ser
1               5

<210> 9
<211> 10
<212> PRT
<213> Artificial Sequence

<220>
<223> SEQ ID NO9 (C-terminus of Ara h 7 isotype 7.0201)

<400> 9
Cys Gly Phe Arg Ser Pro Ser Arg Cys Asp
1               5               10

<210> 10
<211> 13
<212> PRT
<213> Artificial Sequence

<220>
<223> SEQ ID NO10 (C-terminus of Ara h 7 isotype 7.0201)

<400> 10
Gln Asn Cys Gly Phe Arg Ser Pro Ser Arg Cys Asp Leu
1               5               10

<210> 11
<211> 159
<212> PRT
<213> Artificial Sequence

<220>
<223> SEQ ID NO11 (Ara h 7 isotype 7.0101, as expressed in example 1)

<400> 11
Met Ser His His His His His His His Leu Glu Val Leu Phe Gln
1               5                   10                  15
Gly Pro Ser Met Thr Arg Trp Asp Pro Asp Arg Gly Ser Arg Gly Ser
            20              25              30
Arg Trp Asp Ala Pro Ser Arg Gly Asp Asp Gln Cys Gln Arg Gln Leu
            35              40              45
Gln Arg Ala Asn Leu Arg Pro Cys Glu Glu His Met Arg Arg Arg Val
            50              55              60
Glu Gln Glu Gln Glu Gln Glu Gln Asp Glu Tyr Pro Tyr Ser Arg Arg
65              70              75                  80
Gly Ser Arg Gly Arg Gln Pro Gly Glu Ser Asp Glu Asn Gln Glu Gln
            85              90              95
Arg Cys Cys Asn Glu Leu Asn Arg Phe Gln Asn Asn Gln Arg Cys Met
            100             105             110
Cys Gln Ala Leu Gln Gln Ile Leu Gln Asn Gln Ser Phe Trp Val Pro
            115             120             125
Ala Gly Gln Glu Pro Val Ala Ser Asp Gly Glu Gly Ala Gln Glu Leu
            130             135             140
Ala Pro Glu Leu Arg Val Gln Val Thr Lys Pro Leu Arg Pro Leu
145             150             155

<210> 12
<211> 164
<212> PRT
<213> Artificial Sequence

<220>
<223> SEQ ID NO12 (Ara h 7 isotype 7.0201, as expressed in example 1)

<400> 12
Met Ser His His His His His His His His Leu Glu Val Leu Phe Gln
1               5                   10                  15
Gly Pro Ser Met Thr Arg Trp Asp Pro Asp Arg Gly Ser Arg Gly Ser
            20                  25                  30
Arg Trp Asp Ala Pro Ser Arg Gly Asp Asp Gln Cys Gln Arg Gln Leu
        35                  40                  45
Gln Arg Ala Asn Leu Arg Pro Cys Glu Glu His Ile Arg Gln Arg Val
    50                  55                  60
Glu Lys Glu Gln Glu Gln Glu Gln Asp Glu Tyr Pro Tyr Ile Gln Arg
65                  70                  75                  80
Gly Ser Arg Gly Gln Arg Pro Gly Glu Ser Asp Glu Asp Gln Glu Gln
            85                  90                  95
Arg Cys Cys Asn Glu Leu Asn Arg Phe Gln Asn Asn Gln Arg Cys Met
            100                 105                 110
Cys Gln Ala Leu Gln Gln Ile Leu Gln Asn Gln Ser Phe Arg Phe Gln
        115                 120                 125
Gln Asp Arg Ser Gln Leu His Gln Met Glu Arg Glu Leu Arg Asn Leu
        130                 135                 140
Pro Gln Asn Cys Gly Phe Arg Ser Pro Ser Arg Cys Asp Leu Ser Ser
145                 150                 155                 160
Arg Thr Pro Tyr

<210> 13
<211> 158
<212> PRT
<213> Artificial Sequence

<220>
<223> SEQ ID NO13 (Ara h 7 isotype 7.0, as expressed in example 1)

<400> 13
Met Ser His His His His His His His His Leu Glu Val Leu Phe Gln
1               5                   10                  15
Gly Pro Ser Met Thr Arg Trp Asp Pro Asp Arg Gly Ser Arg Gly Leu
            20                  25                  30
Arg Trp Asp Ala Pro Ser Arg Gly Asp Asp Gln Cys Gln Arg Gln Leu
        35                  40                  45
Gln Arg Ala Asn Leu Arg Pro Cys Glu Glu His Ile Arg Gln Arg Val
    50                  55                  60
Glu Gln Glu Gln Glu Gln Glu Gln Asp Glu Tyr Pro Tyr Ser Gln Arg
65                  70                  75                  80
Gly Ser Arg Gly Arg Arg Pro Gly Glu Ser Asp Glu Asp Gln Glu Gln
            85                  90                  95
Arg Cys Cys Asn Glu Leu Asn Arg Phe Gln Asn Asn Gln Arg Cys Met
            100                 105                 110
Cys Gln Ala Leu Gln Gln Ile Leu Gln Asn Gln Ser Phe Arg Phe Gln
        115                 120                 125
Gln Asp Arg Ser Gln Leu His Gln Asn Gly Glu Gly Ala Gln Glu Leu
        130                 135                 140
Ala Pro Glu Leu Arg Val Gln Val Thr Lys Pro Leu Arg Pro
145                 150                 155

<210> 14

<211> 165
<212> PRT
<213> Artificial Sequence

<220>
<223> SEQ ID NO14 (Ara h 2 isotype 2.0201, as expressed in example 1)

<400> 14
Met Ser His His His His His His Ile Glu Gly Arg Thr Met Arg Gln
1               5                   10                  15
Gln Trp Glu Leu Gln Gly Asp Arg Arg Cys Gln Ser Gln Leu Glu Arg
            20                  25                  30
Ala Asn Leu Arg Pro Cys Glu Gln His Leu Met Gln Lys Ile Gln Arg
        35                  40                  45
Asp Glu Asp Ser Tyr Gly Arg Asp Pro Tyr Ser Pro Ser Gln Asp Pro
    50                  55                  60
Tyr Ser Pro Ser Gln Asp Pro Asp Arg Arg Asp Pro Tyr Ser Pro Ser
65                  70                  75                  80
Pro Tyr Asp Arg Arg Gly Ala Gly Ser Ser Gln His Gln Glu Arg Cys
                85                  90                  95
Cys Asn Glu Leu Asn Glu Phe Glu Asn Asn Gln Arg Cys Met Cys Glu
                100                 105                 110
Ala Leu Gln Gln Ile Met Glu Asn Gln Ser Asp Arg Leu Gln Gly Arg
        115                 120                 125
Gln Gln Glu Gln Gln Phe Lys Arg Glu Leu Arg Asn Leu Pro Gln Gln
        130                 135                 140
Cys Gly Leu Arg Ala Pro Gln Arg Cys Asp Leu Glu Val Glu Ser Gly
145                 150                 155                 160
Gly Arg Asp Arg Tyr
                165

<210> 15
<211> 143
<212> PRT
<213> Artificial Sequence

<220>
<223> SEQ ID NO15 (Ara h 6 isotype 6.0101, as expressed in example 1)

<400> 15
Met Ser His His His His His His His His Leu Glu Val Leu Phe Gln
1               5                   10                  15
Gly Pro Ser Met Arg Arg Glu Arg Gly Arg Gln Gly Asp Ser Ser Ser
            20                  25                  30
Cys Glu Arg Gln Val Asp Arg Val Asn Leu Lys Pro Cys Glu Gln His
        35                  40                  45
Ile Met Gln Arg Ile Met Gly Glu Gln Glu Gln Tyr Asp Ser Tyr Asp
    50                  55                  60
Ile Arg Ser Thr Arg Ser Ser Asp Gln Gln Gln Arg Cys Cys Asp Glu
65                  70                  75                  80
Leu Asn Glu Met Glu Asn Thr Gln Arg Cys Met Cys Glu Ala Leu Gln
                85                  90                  95
Gln Ile Met Glu Asn Gln Cys Asp Arg Leu Gln Asp Arg Gln Met Val
                100                 105                 110
Gln Gln Phe Lys Arg Glu Leu Met Asn Leu Pro Gln Gln Cys Asn Phe
        115                 120                 125
Arg Ala Pro Gln Arg Cys Asp Leu Asp Val Ser Gly Gly Arg Cys
    130                 135                 140

<210> 16
<211> 0
<212> PRT
<213> Artificial Sequence

```
<220>
<223> Deleted

<400> 16


<210> 17
<211> 615
<212> PRT
<213> Artificial Sequence

<220>
<223> SEQ ID NO17: Ara h 1.01.01 as used in example 3

<400> 17
Met Ser His His His His His His Ile Glu Gly Arg Thr Met Lys Ser
1               5                   10                  15
Ser Pro Tyr Gln Lys Lys Thr Glu Asn Pro Cys Ala Gln Arg Cys Leu
            20                  25                  30
Gln Ser Cys Gln Gln Glu Pro Asp Asp Leu Lys Gln Lys Ala Cys Glu
        35                  40                  45
Ser Arg Cys Thr Lys Leu Glu Tyr Asp Pro Arg Cys Val Tyr Asp Pro
    50                  55                  60
Arg Gly His Thr Gly Thr Thr Asn Gln Arg Ser Pro Pro Gly Glu Arg
65                  70                  75                  80
Thr Arg Gly Arg Gln Pro Gly Asp Tyr Asp Asp Arg Arg Gln Pro
                85                  90                  95
Arg Arg Glu Glu Gly Gly Arg Trp Gly Pro Ala Gly Pro Arg Glu Arg
            100                 105                 110
Glu Arg Glu Glu Asp Trp Arg Gln Pro Arg Glu Asp Trp Arg Arg Pro
        115                 120                 125
Ser His Gln Gln Pro Arg Lys Ile Arg Pro Glu Gly Arg Glu Gly Glu
    130                 135                 140
Gln Glu Trp Gly Thr Pro Gly Ser His Val Arg Glu Glu Thr Ser Arg
145                 150                 155                 160
Asn Asn Pro Phe Tyr Phe Pro Ser Arg Arg Phe Ser Thr Arg Tyr Gly
                165                 170                 175
Asn Gln Asn Gly Arg Ile Arg Val Leu Gln Arg Phe Asp Gln Arg Ser
            180                 185                 190
Arg Gln Phe Gln Asn Leu Gln Asn His Arg Ile Val Gln Ile Glu Ala
        195                 200                 205
Lys Pro Asn Thr Leu Val Leu Pro Lys His Ala Asp Ala Asp Asn Ile
    210                 215                 220
Leu Val Ile Gln Gln Gly Gln Ala Thr Val Thr Val Ala Asn Gly Asn
225                 230                 235                 240
Asn Arg Lys Ser Phe Asn Leu Asp Glu Gly His Ala Leu Arg Ile Pro
                245                 250                 255
Ser Gly Phe Ile Ser Tyr Ile Leu Asn Arg His Asp Asn Gln Asn Leu
            260                 265                 270
Arg Val Ala Lys Ile Ser Met Pro Val Asn Thr Pro Gly Gln Phe Glu
        275                 280                 285
Asp Phe Phe Pro Ala Ser Ser Arg Asp Gln Ser Ser Tyr Leu Gln Gly
        290                 295                 300
Phe Ser Arg Asn Thr Leu Glu Ala Ala Phe Asn Ala Glu Phe Asn Glu
305                 310                 315                 320
Ile Arg Arg Val Leu Leu Glu Glu Asn Ala Gly Gly Glu Gln Glu Glu
            325                 330                 335
Arg Gly Gln Arg Arg Trp Ser Thr Arg Ser Ser Glu Asn Asn Glu Gly
            340                 345                 350
Val Ile Val Lys Val Ser Lys Glu His Val Glu Glu Leu Thr Lys His
    355                 360                 365
```

```
Ala Lys Ser Val Ser Lys Lys Gly Ser Glu Glu Glu Gly Asp Ile Thr
    370                 375                 380
Asn Pro Ile Asn Leu Arg Glu Gly Glu Pro Asp Leu Ser Asn Asn Phe
385                 390                 395                 400
Gly Lys Leu Phe Glu Val Lys Pro Asp Lys Lys Asn Pro Gln Leu Gln
                405                 410                 415
Asp Leu Asp Met Leu Thr Cys Val Glu Ile Lys Glu Gly Ala Leu
                420                 425                 430
Met Leu Pro His Phe Asn Ser Lys Ala Met Val Ile Val Val Asn
            435                 440                 445
Lys Gly Thr Gly Asn Leu Glu Leu Val Ala Val Arg Lys Glu Gln Gln
        450                 455                 460
Gln Arg Gly Arg Arg Glu Glu Glu Glu Asp Glu Asp Glu Glu Glu Glu
465                 470                 475                 480
Gly Ser Asn Arg Glu Val Arg Arg Tyr Thr Ala Arg Leu Lys Glu Gly
                485                 490                 495
Asp Val Phe Ile Met Pro Ala Ala His Pro Val Ala Ile Asn Ala Ser
                500                 505                 510
Ser Glu Leu His Leu Leu Gly Phe Gly Ile Asn Ala Glu Asn Asn His
            515                 520                 525
Arg Ile Phe Leu Ala Gly Asp Lys Asp Asn Val Ile Asp Gln Ile Glu
        530                 535                 540
Lys Gln Ala Lys Asp Leu Ala Phe Pro Gly Ser Gly Glu Gln Val Glu
545                 550                 555                 560
Lys Leu Ile Lys Asn Gln Lys Glu Ser His Phe Val Ser Ala Arg Pro
                565                 570                 575
Gln Ser Gln Ser Gln Ser Pro Ser Ser Pro Glu Lys Glu Ser Pro Glu
            580                 585                 590
Lys Glu Asp Gln Glu Glu Glu Asn Gln Gly Gly Lys Gly Pro Leu Leu
            595                 600                 605
Ser Ile Leu Lys Ala Phe Asn
    610                 615

<210> 18
<211> 527
<212> PRT
<213> Artificial Sequence

<220>
<223> SEQ ID NO18: Ara h 3.01.01 as used in example 3

<400> 18
Met Ser His His His His His His His His Leu Glu Val Leu Phe Gln
1           5                   10                  15
Gly Pro Ser Met Arg Gln Gln Pro Glu Glu Asn Ala Cys Gln Phe Gln
            20                  25                  30
Arg Leu Asn Ala Gln Arg Pro Asp Asn Arg Ile Glu Ser Glu Gly Gly
        35                  40                  45
Tyr Ile Glu Thr Trp Asn Pro Asn Asn Gln Glu Phe Glu Cys Ala Gly
    50                  55                  60
Val Ala Leu Ser Arg Leu Val Leu Arg Arg Asn Ala Leu Arg Arg Pro
65                  70                  75                  80
Phe Tyr Ser Asn Ala Pro Gln Glu Ile Phe Ile Gln Gln Gly Arg Gly
                85                  90                  95
Tyr Phe Gly Leu Ile Phe Pro Gly Cys Pro Arg His Tyr Glu Glu Pro
            100                 105                 110
His Thr Gln Gly Arg Arg Ser Gln Ser Gln Arg Pro Pro Arg Arg Leu
        115                 120                 125
Gln Gly Glu Asp Gln Ser Gln Gln Arg Asp Ser His Gln Lys Val
    130                 135                 140
His Arg Phe Asp Glu Gly Asp Leu Ile Ala Val Pro Thr Gly Val Ala
145                 150                 155                 160
Phe Trp Leu Tyr Asn Asp His Asp Thr Asp Val Val Ala Val Ser Leu
```

```
                         165                    170                    175
         Thr Asp Thr Asn Asn Asn Asp Asn Gln Leu Asp Gln Phe Pro Arg Arg
                     180                    185                    190
         Phe Asn Leu Ala Gly Asn Thr Glu Gln Glu Phe Leu Arg Tyr Gln Gln
                     195                    200                    205
         Gln Ser Arg Gln Ser Arg Arg Arg Ser Leu Pro Tyr Ser Pro Tyr Ser
                     210                    215                    220
         Pro Gln Ser Gln Pro Arg Gln Glu Glu Arg Glu Phe Ser Pro Arg Gly
         225                    230                    235                    240
         Gln His Ser Arg Arg Glu Arg Ala Gly Gln Glu Glu Glu Asn Glu Gly
                     245                    250                    255
         Gly Asn Ile Phe Ser Gly Phe Thr Pro Glu Phe Leu Glu Gln Ala Phe
                     260                    265                    270
         Gln Val Asp Asp Arg Gln Ile Val Gln Asn Leu Arg Gly Glu Thr Glu
                     275                    280                    285
         Ser Glu Glu Glu Gly Ala Ile Val Thr Val Arg Gly Gly Leu Arg Ile
                     290                    295                    300
         Leu Ser Pro Asp Arg Lys Arg Arg Ala Asp Glu Glu Glu Glu Tyr Asp
         305                    310                    315                    320
         Glu Asp Glu Tyr Glu Tyr Asp Glu Glu Asp Arg Arg Arg Gly Arg Gly
                     325                    330                    335
         Ser Arg Gly Arg Gly Asn Gly Ile Glu Glu Thr Ile Cys Thr Ala Ser
                     340                    345                    350
         Ala Lys Lys Asn Ile Gly Arg Asn Arg Ser Pro Asp Ile Tyr Asn Pro
                     355                    360                    365
         Gln Ala Gly Ser Leu Lys Thr Ala Asn Asp Leu Asn Leu Leu Ile Leu
                     370                    375                    380
         Arg Trp Leu Gly Pro Ser Ala Glu Tyr Gly Asn Leu Tyr Arg Asn Ala
         385                    390                    395                    400
         Leu Phe Val Ala His Tyr Asn Thr Asn Ala His Ser Ile Ile Tyr Arg
                     405                    410                    415
         Leu Arg Gly Arg Ala His Val Gln Val Val Asp Ser Asn Gly Asn Arg
                     420                    425                    430
         Val Tyr Asp Glu Glu Leu Gln Glu Gly His Val Leu Val Val Pro Gln
                     435                    440                    445
         Asn Phe Ala Val Ala Gly Lys Ser Gln Ser Glu Asn Phe Glu Tyr Val
                     450                    455                    460
         Ala Phe Lys Thr Asp Ser Arg Pro Ser Ile Ala Asn Leu Ala Gly Glu
         465                    470                    475                    480
         Asn Ser Val Ile Asp Asn Leu Pro Glu Glu Val Val Ala Asn Ser Tyr
                     485                    490                    495
         Gly Leu Gln Arg Glu Gln Ala Arg Gln Leu Lys Asn Asn Asn Pro Phe
                     500                    505                    510
         Lys Phe Phe Val Pro Pro Ser Gln Gln Ser Pro Arg Ala Val Ala
                     515                    520                    525
```

**Description**

The present invention relates to a diagnostically useful carrier comprising a means for specifically capturing an antibody to Ara h 7 isotype 7.0201 in a sample from a subject; a method comprising the step detecting in a sample from a subject the presence or absence of an antibody to Ara h 7 isotype 7.0201 and a pharmaceutical composition comprising Ara h 7 isotype 7.0201 or a variant thereof.

Among food allergies, those relating to peanut (*Arachis hypogea*) have attracted the most research attention because they are relatively common, typically permanent, and often severe. Food anaphylaxis fatality registries in the United States implicate peanut as a trigger in 59% of 63 deaths and indicate that adolescents and young adults are at greatest risk, with additional risk factors being asthma and delayed injection of epinephrine during anaphylaxis. Peanut is a ubiquitous food, and affected patients, who often react to small doses, are faced with numerous hurdles to avoid ingestion and experience a negative effect on quality of life.

Peanut allergy is primarily suspected in two circumstances: when a patient demonstrates severe allergic reactions or in the context of monitoring allergic conditions to other foods or aeroallergens. When peanut sensitization is discovered by a positive skin prick-test (SPT), or peanut-specific IgE assays, the diagnostic reliability of which relative to clinical disease is currently insufficient, the patient is referred to a specialized center that provides a diagnosis based on a double-blind placebo-controlled food challenge (DBPCFC). This procedure is the gold standard for food allergy diagnosis, but its application requires hospitalization and is costly. Furthermore, even under the control of trained physicians, DBPCFC with peanut may cause severe or even life-threatening reactions.

Therefore, there is a great deal of interest in improved assays for the diagnosis of peanut allergies based on the detection of specific antibodies in samples from subjects suspected of suffering from a peanut allergy.

Assays described in the state of the art rely on the detection of major peanut allergens such as Ara h 1, Ara h 2, Ara h 3 and Ara h 6 using natural or recombinant allergens (Flinterman, A. E., van Hoffen, E., den Hartog, Jager C. F., Koppelman, S., Pasmans, S. G., Hoekstra, M. O., Bruijnzeel-Koomen, C. A., Knulst, A. C., Knol, E. F. (2007): Children with peanut allergy recognize predominantly Ara h 2 and Ara h 6, which remains stable over time, Clin Exp Allergy 2007;37; 1221-1228; de Leon, M. P., Drew, A. C., Glaspole, I. N., Suphioglu, C., O'Hehir, R. E., Rolland, J. M. (2007): IgE cross-reactivity between the major peanut allergen Ara h 2 and tree nut allergens, Mol Immunol 2007; 44: 463-471).

Ara h 2 and Ara h 6 are 2S storage proteins of the conglutin type and belong to the prolamin superfamily. Members of this family are characterized by the presence of a conserved pattern of six or eight cysteine residues located within a sequence of about 100 amino acid residues forming three or four intramolecular disulfide bonds. They are stable to thermal processing and proteolysis.

Ara h 2 is a major allergen of peanuts and occurs in two isoforms with different molecular weights (MWs), which is due to a 12-amino-acid insert with a MW of 1,414 Da. In its primary protein sequence, Ara h 6 exhibits an identity with Ara h 2 of 53% and, in comparison with Ara h 2, possesses ten instead of eight cysteines but no N-glycosylation site. Like Ara h 2, Ara h 6 is resistant to digestion and stable against heating, especially in baking processes. The tertiary structure of Ara h 6 was elucidated and at least four $\alpha$-helical structures and the location parameters of disulfide bonds were identified. In digestion experiments under physiological conditions, Ara h 2 and Ara h 6 form stable fragments even under reducing conditions. This supports the idea that cross-reactivities between Ara h 2 and Ara h 6 are mediated by IgE reactive conformational epitopes (Schmidt, H., Krause, S., Gelhaus, C., Petersen, A., Janssen, O., and Becker, W. M. (2010): Detection and structural characterization of natural Ara h 7, the third peanut allergen of the 2S albumin family, J Proteome Res 2010; 9: 3701-709.).

Ara h 7 is another peanut allergen, and its isotype Ara h 7.0101 was at first identified by the phage display system and cloned, but initially the natural counterpart was not found in peanut extract, which is one of the reasons why its impact on allergy is described in the state of the art as low.

Schmidt et al. (2008, Detection and Structural Characterization of Natural Ara h 7, the Third Peanut Allgergen of the 2S Albumin Family) found that another isotype exists (Ara h 7.0201) Ara h 7.0 is another isotype published in the data base UNIPROT in addition to 7.0101). However, the Schmidt *et al.* do not mention diagnostic applications; theirs is a purely proteomic study.

What is more, only five out of six patients' sera used by Schmidt *et al.*, which had been preselected by Ara h 7.0101 reactivity, showed positive IgE binding with Ara h 7.0201, suggesting that the latter is in fact less (!) reactive and therefore of even lower diagnostic value than Ara h 7.0101. Therefore, Ara h 7.0201 has not been used for routine diagnostics until now as far as the inventors are aware.

In addition, several studies have evaluated a range of immunologic parameters using purified peanut proteins (Ara h 1-3, Ara h 6) and found a correlation of clinical severity with recognition of Ara h 2 and 6 at low concentrations, and Ara h 1 and 3 at higher concentrations, indicating apparent increased potency of Ara h 2 (Peeters, K. A., Koppelman, S. J., van Hoffen, E., van der Tas, C. W., den Hartog, Jager C. F., Penninks, A. H., Hefle, S. L., Bruijnzeel-Koomen, C. A., Knol, E. F., and Knulst, A. C. (2007): Does skin prick test reactivity to purified allergens correlate with clinical severity of peanut allergy? Clin Exp Allergy. 2007 Jan; 37(1):108-15.).

The IgE antibody reactivity to peanut allergens Ara h 1, Ara h 2, Ara h 3, Ara h 6 and Ara h 7 has been investigated in peanut-allergic patients using recombinant allergens as well. Codreanu *et al.* evaluated the performance of the proteins in the diagnosis of peanut allergy in 2 large cohorts of peanut-allergic patients. Measurements of specific IgE were performed using a UniCAP platform with ImmunoCAP tests. They reported that Ara h 2 is the allergen to which peanut-allergic patients are most frequently sensitized, with detectable IgE antibodies being present in 77-100% of cases, followed by Ara h 6 (38-80% cases). By contrast, their data suggest that no patients are monosensitive to Ara h 7 (Codreanu, F., Collignon, O., Roitel, O., Thouvenot, B., Sauvage, C., Vilain, A. C., Cousin, M. O., Decoster, A., Renaudin, JM., Astier, C., Monnez, JM., Vallois, P., Morisset, M., Moneret-Vautrin, D. A., Brulliard, M., Ogier, V., Castelain, M. C., Kanny, G., Bihain, B. E., and Jacquenet, S. (2011): A novel immunoassay using recombinant allergens simplifies peanut allergy diagnosis, Int Arch Allergy Immunol, 2011;154(3):216-26.). The findings that no patients are monosensitive to Ara h 7 has been another reason not to use any isotype of Ara h 7 for routine diagnostics, since it was believed that it does not increase the sensitivity of diagnostic tests for identifying peanut-allergic patients.

At present, a number of patients allergic to peanuts cannot be diagnosed as allergic based on the results of conventional tests. Not in the least in view of the severity of the symptoms associated with peanut allergy, there is continuously increasing demand for diagnostics having the utmost degree of sensitivity.

The problem underlying the present invention is to overcome any shortcomings associated with state of the art diagnostic assays based on the detection of antibodies to peanut allergens.

Another problem underlying the present invention is to provide a new antigen, a new assay, new reagents and a new method that allows for the diagnosis of allergy in patients that remain would be un- or misdiagnosed using conventional assay systems.

Another problem underlying the present invention is to provide an improved antigen for the detection of IgE antibodies in patients suffering from nut allergies, preferably peanut allergies.

Another problem underlying the present invention is to provide an alternative method and alternative reagents for diagnosing nut allergies, preferably peanut allergies.

Another problem underlying the present invention is to provide a method and reagents that, when used in combination with known methods and reagents for the diagnosis of nut allergies, preferably peanut allergies, increase the overall diagnostic reliability, in particular sensitivity.

Another problem underlying the present invention is to provide a method and reagents that, with minimal investment in resources, in particular time and the number of antigens used, allows for the diagnosis of nut allergies, preferably peanut allergies, with the maximum overall diagnostic reliability, in particular sensitivity.

The problem underlying the present invention is solved by the subject-matter of the attached independent and dependent claims.

In a first aspect, the problem underlying the present invention is solved by a diagnostically useful carrier comprising a means for specifically capturing an antibody to Ara h 7 isotype 7.0201, preferably SEQ ID NO6, more preferably to a sequence from the group comprising SEQ ID NO7, SEQ ID NO8, SEQ ID NO9 and SEQ ID NO10, most preferably to SEQ ID NO8, in a sample from a subject.

In another preferred embodiment of the first aspect, the carrier further comprises a means for specifically capturing an antibody to one or more further antigens from the group comprising Ara h 2, Ara h 6, Ara h 1, Ara h 3, Ara h 9, Ara h 8 and Ara h 5, more preferably from the group comprising Ara h 2, Ara h 6, Ara h 1, Ara h 3, Ara h 9, most preferably an antibody to Ara h 2 and/or an antibody to Ara h 6.

In another preferred embodiment of the first aspect, the diagnostically useful carrier is selected from the group comprising a bead, a test strip, a microtiter plate, a blot, preferably from the group comprising western blot, line blot and dot blot, a glass surface, a biochip and a membrane, and is most preferably a microtiter plate or a line blot.

In a second aspect, the problem underlying the present invention is solved by a kit comprising a diagnostically useful carrier comprising a means for specifically capturing an antibody to antigen Ara h 7 isotype 7.0201, preferably SEQ ID NO6 in a sample from a subject, preferably further comprising a means for specifically capturing an antibody to one or more further antigens, more preferably from the group Ara h 2, Ara h 6, Ara h 1, Ara h 3, Ara h 9, Ara h 8 and Ara h 5, more preferably from the group comprising Ara h 2, Ara h 6, Ara h 1, Ara h 3, Ara h 9, most preferably an antibody to Ara h 2 and/or an antibody to Ara h 6, optionally as well as a means for specifically detecting a captured antibody.

In another preferred embodiment of the second aspect, the diagnostically useful carrier further comprises a means for specifically capturing an antibody to one or more further antigens from the group comprising Ara h 2, Ara h 6, Ara h 1, Ara h 3, Ara h 9, Ara h 8 and Ara h 5, wherein the means for specifically capturing an antibody to Ara h 7 isotype 7.0201, preferably SEQ ID NO6, more preferably to a sequence from the group comprising SEQ ID NO7, SEQ ID NO8, SEQ ID NO9 and SEQ ID NO10, most preferably to SEQ ID NO8, and the means for specifically capturing an antibody to one or more further antigens are on separate carriers.

In a preferred embodiment of the second aspect, the diagnostically useful carrier further comprises a means for specifically capturing an antibody to one or more further antigens from the group comprising Ara h 2, Ara h 6, Ara h 1, Ara h 3, Ara h 9, Ara h 8 and Ara h 5, wherein the means for specifically capturing an antibody to Ara h 7 isotype 7.0201, preferably SEQ ID NO6 more preferably to a sequence from the group comprising SEQ ID NO7, SEQ ID NO8, SEQ ID NO9 and SEQ ID NO10, most preferably to SEQ ID NO8, and the means for specifically capturing an antibody to one or more further antigens are on one carrier, preferably covalently linked to one carrier.

In a third aspect, the problem underlying the present invention is solved by a method, preferably for diagnosing or aiding the diagnosis of a nut allergy, more preferably a peanut allergy, comprising the step detecting in a sample from a subject the presence of an antibody to Ara h 7 isotype 7.0201, preferably SEQ ID NO6, more preferably to a sequence from the group comprising SEQ ID NO7, SEQ ID N0NO8, SEQ ID NO9 and SEQ ID NO10, most preferably to SEQ ID NO8.

In a preferred embodiment of the third aspect, the method further comprises detecting in a sample from a subject the presence of an antibody to one or more further antigens, preferably selected from the group comprising Ara h 2, Ara h 6, Ara h 1, Ara h 3, Ara h 9, Ara h 8 and Ara h 5, more preferably from the group comprising Ara h 2, Ara h 6, Ara h 1, Ara h 3 and Ara h 9, most preferably Ara h 2 and/or Ara h 6.

In another preferred embodiment of the third aspect, the presence of an antibody to Ara h 7 isotype 7.0201, preferably SEQ ID NO6 more preferably to a sequence from the group comprising SEQ ID NO7, SEQ ID N0NO8, SEQ ID NO9 and SEQ ID NO10, most preferably to SEQ ID NO8 and the presence of an antibody to one or more further antigens, preferably Ara h 2 and/or Ara h 6, is detected simultaneously.

In another preferred embodiment of the third aspect, the presence of an antibody to Ara h 7 isotype 7.0201, preferably SEQ ID NO6, more preferably to a sequence from the group comprising SEQ ID NO7, SEQ ID N0NO8, SEQ ID NO9 and SEQ ID NO10, most preferably to SEQ ID NO8, and the presence of an antibody to one or more further antigens, preferably Ara h 6 and/or Ara h 2, is detected in spatially separate binding reactions.

In another preferred embodiment of the third aspect, the presence of an antibody to Ara h 7 isotype 7.0201, and the presence of an antibody to one or more further antigens, preferably Ara h 6 and/or Ara h 2, is detected in a one-pot reaction.

In another preferred embodiment of the third aspect, the method comprises the step contacting the diagnostically useful carrier according to the present invention with a sample from the subject.

In another preferred embodiment of any aspect, the subject suffers from or is suspected to suffer from an allergy, preferably an allergy to a nut, more preferably a peanut.

In another preferred embodiment of any aspect, the antibody to Ara h 7 isotype 7.0201, preferably SEQ ID NO6 is an antibody monospecific to Ara h 7 isotype 7.0201, more preferably to a sequence from the group comprising SEQ ID NO7, SEQ ID N0NO8, SEQ ID NO9 and SEQ ID NO10, most preferably to SEQ ID NO8.

In a fourth aspect, the problem underlying the present invention is solved by a pharmaceutical composition comprising SEQ ID NO 8, more preferably a sequence from the group comprising SEQ ID NO6, SEQ ID NO7, SEQ ID NO8, SEQ ID NO9 and SEQ ID NO10, more preferably SEQ ID NO6, most preferably Ara h 7 isotype 7.0201 or a variant thereof and preferably one or more further antigens from the group comprising Ara h 2, Ara h 6, Ara h 1, Ara h 3, Ara h 9, Ara h 8 and Ara h 5 and a variant thereof, more preferably from the group comprising Ara h 2, Ara h 6, Ara h 1, Ara h 3, Ara h 9 and a variant thereof, most preferably Ara h 2 and/or Ara h 6.

In a fifth aspect, the problem underlying the present invention is solved by a polypeptide comprising SEQ ID NO 8, more preferably a sequence from the group comprising SEQ ID NO6, SEQ ID NO7, SEQ ID NO8, SEQ ID NO9 and SEQ ID NO10, more preferably SEQ ID NO6, most preferably Ara h 7 isotype 7.0201, or a variant thereof.

In a preferred embodiment, the polypeptide according to the present invention is immobilized, purified, and/or a fusion protein, preferably purified, more preferably immobilized and purified, more preferably purified and recombinant and immobilized.

In a sixth aspect, the problem is solved by a use of the polypeptide, the carrier or the kit according to the present invention for the diagnosis of peanut allergy, wherein preferably the sensitivity is increased.

In a seventh aspect, the problem is solved by a use of the polypeptide according to the present invention for the manufacture of a kit for the diagnosis of peanut allergy, wherein preferably the sensitivity of the diagnosis is increased.

Preferably the polypeptide comprising SEQ ID NO 8, more preferably a sequence from the group comprising SEQ ID NO6, SEQ ID NO7, SEQ ID NO8, SEQ ID NO9 and SEQ ID NO10, more preferably SEQ ID NO6, most preferably Ara h 7 isotype 7.0201 or a variant thereof is a composition comprising said polypeptide and Ara h 2 and/or Ara h 6 or a variant thereof.

In an 8th aspect, the problem is solved by an antibody, preferably IgE class antibody binding specifically to Ara h 7 isotype 7.0201, preferably SEQ ID NO6, more preferably a sequence from the group comprising SEQ ID NO7, SEQ ID NO8, SEQ ID NO9 and SEQ ID NO10, most preferably to SEQ ID NO8, which is preferably an isolated antibody. The antibody may be isolated by affinity chromatography using standard protocols from a patient sample using the antigen as affinity ligand.

The present invention centers around the detection of an antibody to Ara h 7 isotype 7.0201, more specifically its surprisingly immunoreactive C-terminus, in particular the epitope comprising SEQ ID NO8, as part of a diagnostic method practiced on a sample from a patient suspected of having an allergy to a nut, more surprisingly some allergic patients have antibodies only that are monospecific to Ara h 7 isotype 7.0201 comprising SEQ ID NO8 and do not bind to the other Ara h 7 isotypes, more specifically 7.0101 and Ara h 7.0, let alone Ara h 2 or Arah h 6.

This is in striking contrast to previously published studies, for example the one by Codreanu *et al.*, which suggest that the detection of an antibody to any (!) Ara h 7 isotype has limited diagnostic value compared to or, in particular, beyond data resulting from the detection of Ara h 2 and/or Ara h 6. In particular, these studies suggest that there are no patients having antibodies monospecific to any Ara h 7 isotype.

The invention relates to a diagnostically useful carrier, which is preferably a solid carrier for contacting a means for specifically capturing an antibody, which means is associated with said carrier, with a bodily fluid sample from a subject, preferably a mammalian subject, more preferably a human subject. In a preferred embodiment the solid carrier is a diagnostic device, more preferably selected from the group comprising a bead, a test strip, a microtiter plate, blot, a glass surface, a biochip and a membrane, more preferably from the group comprising a blot, a test strip and a membrane. In a most preferred embodiment, the diagnostically useful carrier is a microtiter plate or line blot (Raoult, D., and Dasch, G. A. (1989), The line blot: an immunoassay for monoclonal and other antibodies. Its application to the serotyping of gram-negative bacteria. J. Immunol. Methods, 125 (1-2), 57-65; WO2013041540). In a preferred embodiment, the term "line blot", as used herein refers to a test strip, more preferably membrane-based, that has been coated with one or more means for capturing an antibody, preferably a polypeptide each. If two or more means are used, they are preferably spatially separated on the carrier. Preferably, the width of the bands is at least 30, more preferably 40, 50, 60, 70 or 80 % the width of the test strip. The test strip may comprise one or more control bands for confirming that it has been contacted with sample sufficiently long and under adequate conditions, in particular in the presence of human serum, antibody conjugate, or both. A multitude of line blots are commercially available, for example from EUROIMMUN, Lübeck, Germany.

The sample from a subject used to practice the present invention comprises antibodies, also referred to as immunoglobulins. Typically the sample is a bodily fluid comprising a representative set of the entirety of the subject's immunoglobulins. However, the sample, once provided, may be subjected to further processing which may include fractionation, centrifugation, enriching or isolating the entirety of immunoglobulins or any immunoglobulin class of the subject, preferably IgE, which may affect the relative distribution of immunoglobulins of the various classes. The sample may be selected from the group comprising whole-blood, serum, cerebrospinal fluid and saliva and is preferably serum. In a most preferred embodiment, the sample comprises IgE class antibodies.

The diagnostically useful carrier comprises a means for specifically capturing an antibody to Ara h 7 isotype 7.0201, preferably to SEQ ID NO8, optionally in combination with one or more further antigens such as Ara h 2 and/or Ara h 6. In a preferred embodiment, the terms "Ara h 7 isotype 7.0201", "Ara h 2", "Ara h 6", "Ara h 1", "Ara h 3", "Ara h 5", "Ara h 8" and "Ara h 9", as used herein, refer to the polypeptides represented by data base codes B4XID4 (Ara h 7 Isotype 7.0201, expressed sequence without signal peptide: SEQ ID NO2) and, Q6PSU2 (Ara h 2, expressed sequence without signal peptide: SEQ ID NO4), Q647G9 (Ara h 6, expressed sequence without signal peptide: SEQ ID NO5), P43238 (Ara h 1), 082580 (Ara h 3), Q9SQI9 (Ara h 5), B0YIU5 or Q6VT83 (two isotypes of Ara h 8), and B6CG41 or B6CEX8 (two isotypes of Ara h 9) and variants thereof, respectively. Preferably the term "Ara h 7", as used herein, refers to the entirety of the three Ara h 7 isotypes, the term "Ara h 7.0101" refers to the isotype having the sequence SEQ ID NO1, and the term "Ara h 7.0" refers to the isotype having the sequence SEQ ID NO3. Any data base codes referred to throughout this application refers to the polypeptide sequence available via the NCBI data bases as online at the priority date of this application. Preferably antibodies are detected in the sample that bind to Ara h 7.0201 according to the present invention, but not to any of the other Ara h 7 isotypes, more specifically Ara h 7.0101 and Ara h 7.0, and the means for specifically capturing an antibody to Ara h 7 isotyp 7.0201 is configured for this purpose and is preferably a polypeptide comprising a sequence selected from the group comprising SEQ ID NO8 or a variant thereof, preferably a sequence from the group comprising SEQ ID NO7, SEQ ID NO8, SEQ ID NO6, SEQ ID NO9 and SEQ ID NO10, more preferably SEQ ID NO6, most preferably Ara h 7 isotype 7.0201 or a variant thereof.

Preferably the antibody to Ara h 7 isotype 7.0201 is an antibody to SEQ ID NO6, more preferably a sequence from the group comprising SEQ ID NO7, SEQ ID NO8, SEQ ID NO9 and SEQ ID NO10, most preferably SEQ ID NO8. More preferably, it does not bind to any epitopes shared by the three Ara h 7 isotypes.

In a preferred embodiment, the term "a means for specifically capturing an antibody to [antigen] X and an antibody to [antigen] Y", as used herein, refers to the sum of a means that specifically captures an antibody to [antigen] X, but not one to [antigen] Y, and a means that specifically captures an antibody to [antigen] Y, but not one to [antigen] X.

For example, X could be Ara h 2 and Y could be Ara h 6. In this case, a means for specifically capturing an antibody to Ara h 2 and Ara h 6 would comprise a means for specifically capturing an antibody to Ara h 2 and, in addition, a means for specifically capturing an antibody to Ara h 6. For instance, a line blot coated with Ara h 2 and Ara h 6, spatially separated from each other, is a means for specifically capturing an antibody to Ara h 2 and Ara h 6.

According to the present invention, the carrier comprises one or more means for specifically capturing an antibody, preferably one or more, more preferably two or more, more preferably three or more, more preferably four or more such means, each of them capable of specifically capturing a different antibody. In a most preferred embodiment, the carrier comprises a means for specifically detecting an antibody to Ara h 2, Ara h 7 isotype 7.0201 and Ara h 6. Said means is preferably immobilized on said carrier. In a preferred embodiment, the means for specifically capturing an antibody is a polypeptide comprising or consisting of an antigen such as from the group comprising Ara h 2, Ara h 7 isotype 7.0201 and Ara h 6, preferably Ara h 7 isotype 7.0201, or a variant thereof. Preferably said polypeptide comprises a sequence from the group comprising SEQ ID NO8, more preferably a sequence from the group comprising SEQ ID NO7, SEQ ID NO8, SEQ ID NO9 and SEQ ID NO10, more preferably SEQ ID NO6, most preferably Ara h 7 isotype 7.0201 or a variant thereof. The polypeptide may be a linear peptide or a folded polypeptide, the latter preferably a variant adopting essentially the same fold as Ara h 7 isotype 7.0201 as may be determined by CD spectroscopy. In a preferred embodiment, the peptide or polypeptide comprises an epitope to the antibody to be captured or detected of at least 7, preferably 10, more preferably 15 amino acids. Said antigen, together with the insoluble carrier to which it is attached, may be separated from a reaction mixture, wherein it is contacted with a sample, in a straightforward manner, for example by filtration, centrifugation or decanting. Said antigen may be immobilized in a reversible or irreversible manner. For example, the immobilization is reversible if the molecule interacts with the carrier via ionic interactions which may be masked by addition of a high concentration of salt or if the molecule is bound via a cleavable covalent bond. By contrast, the immobilization is irreversible if the molecule is tethered to the carrier via a covalent bond that cannot be cleaved in aqueous solution. The polypeptide may be indirectly immobilized, for example by immobilizing an antibody or other entity having affinity to the polypeptide, followed by addition of the polypeptide and formation of a polypeptide-antibody complex.

If the diagnostically useful carrier is a bead, a mixture of beads, each carrying one type means for specifically capturing an antibody may be used. The mixture comprise at least one bead carrying a means for specifically capturing an antibody to Ara h 7 isotype 7.0201, preferably to a sequence from the group comprising SEQ ID NO6, more preferably a sequence from the group comprising SEQ ID NO7, SEQ ID NO8, SEQ ID NO9 and SEQ ID NO10, most preferably SEQ ID NO8. In addition, the mixture of beads may comprise at least one additional bead, each bead carrying a means for specifically capturing an antibody to one or more from the group comprising Ara h 2, Ara h 6, Ara h 1, Ara h 3, Ara h 9, Ara h 8 and Ara h 5 and a variant thereof, more preferably from the group comprising Ara h 2, Ara h 6, Ara h 1, Ara h 3, Ara h 9 and a variant thereof, most preferably Ara h 2 and/or Ara h 6.

If the diagnostically useful carrier is a bead, the bead may alternatively comprise, in addition to a means for specifically capturing an antibody to Ara h 7 isotype 7.0201, preferably to a sequence from the group comprising SEQ ID NO6, more preferably a sequence from the group comprising SEQ ID NO7, SEQ ID NO8, SEQ ID NO9 and SEQ ID NO10, most preferably SEQ ID NO8, at least one more additional means for specifically capturing an antibody, which may be an antibody to one or more from the group comprising Ara h 2, Ara h 6, Ara h 1, Ara h 3, Ara h 9, Ara h 8 and Ara h 5 and a variant thereof, more preferably from the group comprising Ara h 2, Ara h 6, Ara h 1, Ara h 3, Ara h 9 and a variant thereof, most preferably Ara h 2 and/or Ara h 6. Most preferably, such a bead comprises a means for for specifically capturing an antibody to Ara h 7 isotype 7.0201, preferably to a sequence from the group comprising SEQ ID NO6, more preferably a sequence from the group comprising SEQ ID NO7, SEQ ID NO8, SEQ ID NO9 and SEQ ID NO10, most preferably SEQ ID NO8, a means for specifically capturing an antibody to Ara h 2 and a means for specifically capturing an antibody to Ara h 6.

However, the teachings of the present invention may not only be carried out using polypeptides, for example Ara h 7 isotype 7.0201, optionally in combination with one or more further antigens such as Ara h 2 and/or Ara h 6, having the exact sequences referred to in this application explicitly, for example by function, name, sequence or accession number, or implicitly, but also using variants of such polypeptides.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 10, 15, 25, 50, 75, 100, 150 or 200 successive amino acids of the original sequence or a variant thereof. The total length of the variant may be at 25, 30, 40, 50, 60, 70, 80, 90, 100 or more amino acids.

In another preferred embodiment, the term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

In a preferred embodiment, variants may, in addition, comprise chemical modifications, for example isotopic labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or variants thereof.

The variant of the polypeptide has biological activity. In a preferred embodiment such biological activity is the ability to bind to the respective antibody. For example, a variant of Ara h 7 isotype 7.0201 has the ability to bind specifically to an IgE antibody to Ara h 7 isotype 7.0201 in a sample obtained from a subject allergic to peanut, since it comprises epitopes to which said antibody binds. Preferably it comprises an epitope recognized by an antibody in a sample from an allergic patient, which antibody binds to Ara h 7 isotype 7.0201, more preferably to an epitope comprising SEQ ID NO8, most preferably to SEQ ID NO8, and more preferably does not bind not to isotypes 7.0101 or 7.0). The person skilled in the art is capable of designing variants having biological activity by starting from the original Ara h 7 isotype 7.0201 sequence, bearing in mind the importance of SEQ ID NO8, introduce modifications such as point mutations, truncations and the like and subsequently confirm that the variant still has biological activity by testing whether said variant binds to an IgE antibody to Ara h 7 isotype 7.0201 in a sample obtained from a subject allergic to peanut.

If a polypeptide is used as the means for specifically capturing an antibody, for example to Ara h 7 isotype 7.0201, said polypeptide, when used to carry out the teachings of the present invention, may be provided in any form and at any degree of purification, from tissues, fruits or cells comprising said polypeptide in an endogenous form, more preferably cells overexpressing the polypeptide, crude or enriched lysates of such cells, to purified and/or isolated polypeptide which may be essentially pure. In a preferred embodiment, the polypeptide is a native polypeptide, wherein the term "native polypeptide", as used herein, refers to a folded polypeptide, more preferably to a folded polypeptide purified from tissues or cells, more preferably from mammalian cells or tissues, optionally from non-recombinant tissues or cell. If a native polypeptide is used, it is preferably enriched compared to its natural state.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, *i.e.* at least 60, 70, 80, 90, 95 or 99 percent of the polypeptide in the respective sample consists of said polypeptide as judged by SDS polyacrylamide gel electrophoresis followed by Coomassie blue staining and visual inspection. Preferably any polypeptide on a carrier used as a means to capture an antibody is pure.

The subject according to the present invention providing the sample is an organism producing antibodies, preferably IgE class or equivalent allergy-related antibodies, more preferably from a mammal, most preferably a human.

Within the scope of the present invention is a diagnostically useful carrier comprising a means for specifically capturing an antibody to an antigen such as Ara h 7 isotype 7.0201. In a preferred embodiment, the term "specifically capturing an antibody", as used herein, refers to the ability to bind specifically to the antibody of interest to the effect that it is bound and removed from the sample, whereas other antibodies, preferably of the same class, are essentially not bound and remain in the sample.

The diagnostically useful carrier according to the invention serves as a scaffold for the one or more means for specifically capturing an antibody, preferably a diagnostically relevant antibody. Said carrier is suitable for carrying out a diagnostic method. By using a carrier rather than free, soluble means for specifically capturing an antibody, it is more straightforward to isolate and separate from the sample a complex comprising the means and the antibody and to wash said complex, for example for the purpose of removing any molecules binding non-specifically to the means, complex or carrier. In a preferred embodiment, the diagnostically useful carrier is a diagnostic device, preferably selected from the group comprising a bead, a test strip, a microtiter plate, a blot and a membrane, and is preferably a microtiter plate or line blot.

According to the present invention, a means for specifically detecting a captured antibody is provided, optionally as part of a kit. In a preferred embodiment, the term "specifically detecting a captured antibody", as used herein, means that the antibody binding specifically to the means for specifically capturing the antibody, preferably Ara h 7 isotype 7.0201, following capture, is detected rather than any other antibody present in the sample. In a preferred embodiment, the term "binding specifically", as used herein, means that the binding is stronger than a binding reaction characterized by a dissociation constant of $1 \times 10^{-5}$ M, more preferably $1 \times 10^{-7}$ M, more preferably $1 \times 10^{-8}$ M, more preferably $1 \times$

$10^{-9}$ M, more preferably $1 \times 10^{-10}$ M, more preferably $1 \times 10^{-11}$ M, more preferably $1 \times 10^{-12}$ M, as determined by surface plasmon resonance using Biacore equipment at 25 °C in PBS buffer at pH 7.

In a preferred embodiment, the means for specifically capturing an antibody to Ara h 7 isotype 7.0201 and the means for specifically capturing an antibody to one or more further antigens are on separate carriers. This means that the means are not attached to a single carrier, but one or more carriers that are separate and/or separable without damaging them. For example, the means for specifically capturing an antibody to Ara h 7 isotype 7.0201 may be attached to a first test strip, and the means for specifically capturing an antibody to Ara h 2 is attached to another test strip which is separate from the first test strip.

In another preferred embodiment, the means for specifically capturing an antibody to Ara h 7 isotype 7.0201 and the means for specifically capturing an antibody to one or more further antigens are on one, preferably covalently linked to one carrier. This means that the means are attached to one carrier which may not be disassembled, without damaging the carrier, such that the means are on separate carriers. For example, the means may be all coated on one test strip, particular in the form of a line blot.

The inventive teachings provide a kit, preferably for diagnosing an allergy, more preferably for diagnosing a peanut allergy. Such a kit is a container that comprises specific reagents required to practice the inventive method, in particular the diagnostically useful carrier according to the present invention, optionally in addition to one or more solutions required to practice the inventive method, preferably selected from or all from the group comprising sample dilution buffer, washing buffer and buffer comprising a means for detecting any specifically captured antibody, such as a secondary antibody and optionally a means for detecting the latter. Furthermore, it may comprise instructions detailing how to use the kit and the inventive diagnostically useful carrier for contacting the inventive polypeptide with a bodily fluid sample from a subject, preferably a human subject, for example a line blot, wherein the inventive means for specifically capturing an antibody to Ara h 7 isotype 7.0201, is immobilized on the line blot. Furthermore, the kit may comprise a positive control, for example a recombinant antibody known to bind to Ara h 7 isotype 7.0201, and a negative control, for example a protein having no detectable affinity to Ara h 7 isotype 7.0201 such as bovine serum albumin. Finally, such a kit may comprise a standard solution comprising an Ara h 7 isotype 7.0201-binding antibody for preparing a calibration curve. In a preferred embodiment, the kit comprises a device, preferably a blot-based device such as a line blot coated with a means for specifically capturing an antibody to Ara h 7 isotype 7.0201 and, optionally, an antibody to one or more further antigens such as Ara h 2 and/or Ara h 6.

According to the invention, a means for detecting the one or more captured antibodies is required and may be included in a kit. The person skilled in the art is aware of many methods that may be used, which are also described in the state of the art, for example in Zane, H. D. (2001), Immunology - Theoretical & Practical Concepts in Laboratory Medicine, W. B. Saunders Company, in particular in Chapter 14. In a preferred embodiment, a secondary antibody binding to the constant region of the one or more captured antibodies, which is the corresponding primary antibody, is used, which secondary antibody may be associated with a label that is straightforward to detect, for example a fluorescent, radioactive or enzymatically active label, the latter of which may catalyze a chemiluminescent reaction or the generation of a molecule detectable using colorimetry or spectroscopy or another analytical method.

Alternatively, a biological functional assay may be used as a means for detecting the one or more captured antibody under the proviso that it is an IgE class antibody, preferably an assay based on basophil activation by IgE antibody. Such assays have been described in the state of the art, for example Hausmann, O. V., Gentinetta, T., Bridts, C. H., and Ebo, E. G. (2009): The Basophil Activation Test in Immediate-Type Drug Allergy, Immunol. Allergy Clin. N. Am. 29, 555-566.

In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a certain treatment, for example the administration of suitable drugs such as drugs for the desensitization of allergic patients. In other words, the term "diagnosis" comprises not only diagnosing, but also prognosticating and/or monitoring the course of a disease or disorder.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. The term "diagnosis" may also refer to a method or agent used to choose the most promising treatment regime for a patient. In other words, the method or agent may relate to selecting a treatment regimen for a subject.

The present invention relates to a method comprising the step detecting in a sample from a subject the presence or absence of an antibody to Ara h 7 isotype 7.0201, preferably to a sequence from the group comprising SEQ ID NO6, more preferably a sequence from the group comprising SEQ ID NO7, SEQ ID NO8, SEQ ID NO9 and SEQ ID NO10, most preferably SEQ ID NO8. Such a method may comprise the steps a) providing a sample from a subject, b) contacting the sample with the diagnostically useful carrier according to the present invention under conditions compatible with the formation of a complex comprising the diagnostically useful carrier and the antibody, more specifically the means for specifically capturing the antibody and the antibody, c) isolating any said complex, for example by removing the sample, d) optionally washing said complex, and e) detecting said complex. The method is preferably an *in vitro* method. The detection of the complex for the prognosis, diagnosis, methods or test kit according to the present invention comprises the use of a method selected from the group comprising immunodiffusion techniques, basophil activation by IgE antibody, immunoelectrophoretic techniques, light scattering immunoassays, agglutination techniques, labeled immunoassays such as those from the group comprising radiolabeled immunoassay, enzyme immunoassays such as colourimetric assays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14.

In many cases detecting the absence or presence of an antibody, optionally meaning determining whether the concentration of the antibody is beyond a certain threshold preferably as set by measurement using ELISA in the implicit detection limit by this method, often suggested by the detection limit, in the sample, is sufficient for the diagnosis. If the antibody can be detected, this will be information instrumental for the clinician's diagnosis and indicates an increased likelihood that the patient suffers from a disease. In a preferred embodiment, the relative concentration of the antibody in the serum, compared to the level that may be found in the average healthy subject, may be determined. In a preferred embodiment, the term "detecting the presence", as used herein, means that it is sufficient to check whether a signal sufficiently beyond any background level may be detected using a suitable complex detection method that indicates that the antibody of interest is present or more antibody of interest is present than would be in a healthy subject. In a more preferred embodiment this may involve determining whether the concentration is at least 0.1, preferably 0.2, 0.5, 1, 2, 5, 10, 20, 25, 50, 100, 200, 500, 1000, 10000 or 100000 times higher than the concentration of the antibody of interest found in the average healthy subject.

In a preferred embodiment, the absence or presence of at least antibodies, such as an antibody to Ara h 7 isotype 7.0201 and the antibody to Ara h 2 and/or the antibody to Ara h 6, is detected simultaneously, *i.e.* at the same time. This is convenient in terms of efficient diagnostic procedures, as a maximum of diagnostic information is obtained in a given period of time. Of course a prerequisite is that sufficient capacity is available for running all reactions.

In a preferred embodiment, the absence or presence of at least two antibodies, such as an antibody to Ara h 7 isotype 7.0201 and the antibody to Ara h 2 and/or the antibody to Ara h 6, is detected in spatially separate reactions. This means that these reactions run in different reaction mixtures in separate vessels.

If more than one antibody is to be detected, the method may be carried out in a one-pot reaction. Preferably, the term "one-pot reaction", as used herein, means that two or more, preferably all reactions carried out for the purpose of detecting the presence or absence of an antibody are carried out in the same reaction mixture in one reaction vessel, without physical barriers between the reactions, by contrast to experimental settings contemplating that at least two reactions are carried out in separate solutions and reaction vessels.

The antibody to be detected may be a monospecific antibody. In a preferred embodiment, the term "monospecific antibody", as used herein, refers to an antibody binding to one antigen only, preferably one diagnostically relevant antigen only, more preferably only one diagnostically relevant antigen from the group comprising Ara h 7, Ara h 2 and Ara h 6. For example, a monospecific antibody may bind to Ara h 7 isotype 7.0201, preferably to SEQ ID NO6, more preferably to a sequence from the group comprising SEQ ID NO7, SEQ ID NO8, SEQ ID NO9 and SEQ ID NO10, most preferably to SEQ ID NO8, but not any other allergen such as isotypes Ara h 70101, Ara h 7.0, or such as Ara h 2 and/or Ara h 6. If a patient has a monospecific antibody only, their allergy can only be detected, by way of serology, if a means for specifically capturing and detecting said monospecific antibody is used. If a diagnostic assay based on means to detect antibodies to Ara h 7 isotypes other than 7.0201, which do not comprise SEQ ID NO8, are used, the result of the assay may be false-negative, since the antibody monospecific to Ara h 7 isotype 7.0201, more preferably SEQ ID NO8, cannot be detected.

The invention provides a use of a means for specifically capturing an antibody to Ara h 7 isotype 7.0201, which is preferably a polypeptide comprising Ara h 7 isotype 7.0201 or variant thereof, for increasing the sensitivity of a diagnostically useful carrier or a method for diagnosing a nut allergy, preferably a peanut allergy, optionally in combination with a means for specifically capturing an antibody to one or more further antigens such as Ara h 2 and/or Ara h 6.

The invention provides a pharmaceutical composition, preferably a vaccine, comprising Ara h 7 isotype 7.0201, preferably a polypeptide comprising SEQ ID NO6, more preferably a sequence from the group comprising SEQ ID NO7, SEQ ID NO8, SEQ ID NO9 and SEQ ID NO10, most preferably SEQ ID NO8, or a variant thereof, optionally in combination with one or more further antigens such as Ara h 2 and/or Ara h 6 or a variant thereof, which composition is preferably suitable for administration to a subject, preferably a mammalian subject, more preferably to a human. Such a pharmaceutical composition may comprise a pharmaceutically acceptable carrier. The pharmaceutical composition may, for example, be administered orally, parenterally, by inhalation spray, topically, by eyedrops, rectally, nasally, buccally, vaginally or via an implanted reservoir, wherein the term "parentally", as used herein, comprises subcutaneous, intracutaneous, intravenous, intramuscular, intra-articular, intrasynovial, instrasternal, intrathecal, intralesional and intracranial injection or infusion techniques. The pharmaceutical composition may be provided in suitable dosage forms, for example capsules, tablets and aqueous suspensions and solutions, preferably in sterile form. It may be used in a method of treatment of a disease, preferably an allergy, which method comprises administering an effective amount of the inventive polypeptide to a subject. A hypoallergenic variant of Ara h 7 and, optionally, one or more further antigens such as Ara h 2 and/or Ara h 6 may be used. The person skilled in the art is familiar with methods for the generation of hypoallergenic variants of known allergens.

The invention provides a vaccine comprising Ara h 7 isotype 7.0201, more preferably a polypeptide comprising SEQ ID NO6, more preferably a sequence from the group comprising SEQ ID NO7, SEQ ID NO8, SEQ ID NO9 and SEQ ID NO10, most preferably SEQ ID NO8.

The invention provides a method for treating, preventing or ameliorating an allergy, preferably a nut allergy, more preferably a peanut allergy, but administering to a subject the inventive pharmaceutical composition.

The invention provides a use of Ara h 7 isotype 7.0201, more preferably a polypeptide comprising SEQ ID NO8, more preferably a sequence from the group comprising SEQ ID NO7, SEQ ID NO6, SEQ ID NO9 and SEQ ID NO10, more preferably SEQ ID NO6, most preferably Ara h 7 isotype 7.0201 or variant thereof, preferably in combination with Ara h 2 and/or Ara h 6 or a variant thereof, for the manufacture of a kit for the diagnosis of peanut allergy, in which a diagnostic assay with an increased sensitivity is provided. Such manufacture may relate to a method comprising the step immobilizing on a diagnostically useful carrier Ara h 7 isotype 7.0201, more preferably a polypeptide comprising SEQ ID NO6, more preferably a sequence from the group comprising SEQ ID NO7, SEQ ID NO8, SEQ ID NO9 and SEQ ID NO10, most preferably SEQ ID NO8, preferably in combination with Ara h 2 and/or Ara h 6 or a variant thereof.

The invention provides a use of Ara h 7 isotype 7.0201, more preferably a polypeptide comprising SEQ ID NO8, more preferably a sequence from the group comprising SEQ ID NO7, SEQ ID NO6, SEQ ID NO9 and SEQ ID NO10, more preferably SEQ ID NO6, most preferably Ara h 7 isotype 7.0201 or variant thereof, preferably in combination with Ara h 2 and/or Ara h 6 or a variant thereof, for the manufacture of a medicament, preferably a vaccine, which is preferably for preventing, amelioratin or treating allergic conditions, in particular a nut allergy, more specifically a peanut allergy.

**Fig. 1** shows IgE binding to the Ara h 7 isoforms of peanut sensitized patients detected by means of ELISA as described in example 1.

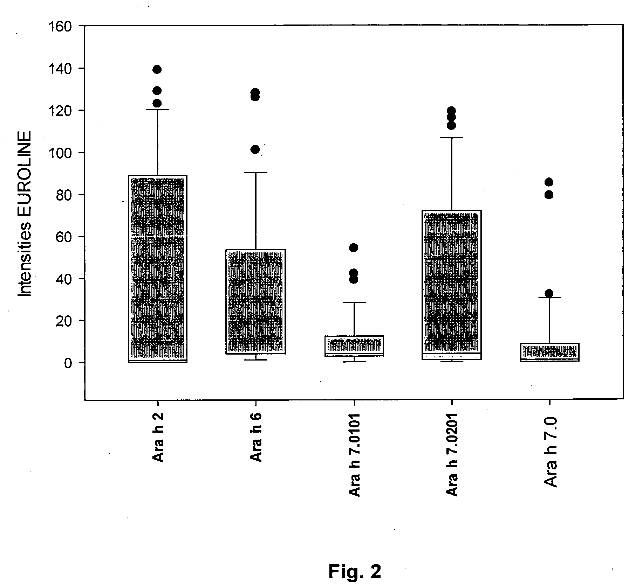

**Fig. 2** shows IgE binding to the Ara h 7 isoforms as well as Ara h 2 and Ara h 6 of peanut-sensitized patients detected by means of line blot as described in example 2.

**Fig. 3** shows a Venn diagram illustrating co-sensitization to 2S albumins Ara h 2, 6 and 7. IgE antibodies were measured by means of a line blot as described in sample 3.

**Fig. 4** shows inhibition experiments described in example 3. Maximum inhibition observed for the five peanut components in patients sensitized to the allergen that was immobilized. Horizontal line marks the median value. The grey, open markers indicate inhibition with the same allergen as the one immobilized.

**Fig. 5** shows the results of epitope mapping of serum IgE against linear peptides of Ara h 7.0201 C-terminus. The normalized signal-to-noise ratio (z-score) is presented for every peptide on the microarray. Positive binding requires at least 6 subsequent peptides yielding a z-score above 3.

**Sequences:**

The present invention comprises a range of novel polypeptides, more specifically

**SEQ ID NO1 (expressed sequence without signal sequence of Ara h 7.0101)**

TRWDPDRGSRGSRWDAPSRGDDQCQRQLQRANLRPCEEHMRRRVEQEQEQEQDEYPY
SRRGSRGRQPGESDENQEQRCCNELNRFQNNQRCMCQALQQILQNQSFWVPAGQEPVA
SDGEGAQELAPELRVQVTKPLRPL

**SEQ ID NO2 (expressed sequence without signal sequence of Ara h 7.0201)**

TRWDPDRGSRGSRWDAPSRGDDQCQRQLQRANLRPCEEHIRQRVEKEQEQEQDEYPYI
QRGSRGQRPGESDEDQEQRCCNELNRFQNNQRCMCQALQQILQNQSFRFQQDRSQLHQ
MERELRNLPQNCGFRSPSRCDLSSRTPY

**SEQ ID NO3 (expressed sequence without signal sequence of Ara h 7.0)**

TRWDPDRGSRGLRWDAPSRGDDQCQRQLQRANLRPCEEHIRQRVEQEQEQEQDEYPYS
QRGSRGRRPGESDEDQEQRCCNELNRFQNNQRCMCQALQQILQNQSFRFQQDRSQLHQ
NGEGAQELAPELRVQVTKPLRP

**SEQ ID NO4 (expressed sequence without signal sequence of Ara h 2.0201)**

RQQWELQGDRRCQSQLERANLRPCEQHLMQKIQRDEDSYGRDPYSPSQDPYSPSQDPD
RRDPYSPSPYDRRGAGSSQHQERCCNELNEFENNQRCMCEALQQIMENQSDRLQGRQQ
EQQFKRELRNLPQQCGLRAPQRCDLEVESGGRDRY

**SEQ ID NO5 (expressed sequence without signal sequence of Ara h 6.0101)**

MRRERGRQGDSSSCERQVDRVNLKPCEQHIMQRIMGEQEQYDSYDIRSTRSSDQQQRCC
DELNEMENTQRCMCEALQQIMENQCDRLQDRQMVQQFKRELMNLPQQCNFRAPQRCDL
DVSGGRC

**SEQ ID NO6 (C-terminus of Ara h 7 isotype 7.0201)**
HQMERELRNLPQNCGFRSPSRCDLSSRTPY

**SEQ ID NO7 (C-terminus of Ara h 7 isotype 7.0201)**
NCGFRSPSRC

**SEQ ID NO8 (reactive epitope from C-terminus of Ara h 7 isotype 7.0201)**
GFRSPS

**SEQ ID NO9 (C-terminus of Ara h 7 isotype 7.0201)**
CGFRSPSRCD

**SEQ ID NO10 (C-terminus of Ara h 7 isotype 7.0201)**
QNCGFRSPSRCDL

**SEQ ID NO11 (Ara h 7 isotype 7.0101, as expressed in example 1)**

MSHHHHHHHHLEVLFQGPSMTRWDPDRGSRGSRWDAPSRGDDQCQRQLQRANLRPCE
EHMRRRVEQEQEQEQDEYPYSRRGSRGRQPGESDENQEQRCCNELNRFQNNQRCMCQ
ALQQILQNQSFWVPAGQEPVASDGEGAQELAPELRVQVTKPLRPL

**SEQ ID NO12 (Ara h 7 isotype 7.0201, as expressed in example 1)**

MSHHHHHHHHHLEVLFQGPSMTRWDPDRGSRGSRWDAPSRGDDQCQRQLQRANLRPCE
EHIRQRVEKEQEQEQDEYPYIQRGSRGQRPGESDEDQEQRCCNELNRFQNNQRCMCQAL
QQILQNQSFRFQQDRSQLHQMERELRNLPQNCGFRSPSRCDLSSRTPY

SEQ ID N013 (Ara h 7 isotype 7.0, as expressed in example 1)

MSHHHHHHHHHLEVLFQGPSMTRWDPDRGSRGLRWDAPSRGDDQCQRQLQRANLRPCEE
HIRQRVEQEQEQEQDEYPYSQRGSRGRRPGESDEDQEQRCCNELNRFQNNQRCMCQAL
QQILQNQSFRFQQDRSQLHQNGEGAQELAPELRVQVTKPLRP

SEQ ID NO14 (Ara h 2 isotype 2.0201, as expressed in example 1)

MSHHHHHHIEGRTMRQQWELQGDRRCQSQLERANLRPCEQHLMQKIQRDEDSYGRDPY
SPSQDPYSPSQDPDRRDPYSPSPYDRRGAGSSQHQERCCNELNEFENNQRCMCEALQQI
MENQSDRLQGRQQEQQFKRELRNLPQQCGLRAPQRCDLEVESGGRDRY

SEQ ID NO15 (Ara h 6 isotype 6.0101, as expressed in example 1)

MSHHHHHHHHHLEVLFQGPSMRRERGRQGDSSSCERQVDRVNLKPCEQHIMQRIMGEQEQ
YDSYDIRSTRSSDQQQRCCDELNEMENTQRCMCEALQQIMENQCDRLQDRQMVQQFKRE
LMNLPQQCNFRAPQRCDLDVSGGRC

SEQ ID NO17: Ara h 1.01.01 as used in example 3

MSHHHHHHIEGRTMKSSPYQKKTENPCAQRCLQSCQQEPDDLKQKACESRCTKLEYDPR
CVYDPRGHTGTTNQRSPPGERTRGRQPGDYDDDRRQPRREEGGRWGPAGPREREREE
DWRQPREDWRRPSHQQPRKIRPEGREGEQEWGTPGSHVREETSRNNPFYFPSRRFSTR
YGNQNGRIRVLQRFDQRSRQFQNLQNHRIVQIEAKPNTLVLPKHADADNILVIQQGQATVTV
ANGNNRKSFNLDEGHALRIPSGFISYILNRHDNQNLRVAKISMPVNTPGQFEDFFPASSRDQ
SSYLQGFSRNTLEAAFNAEFNEIRRVLLEENAGGEQEERGQRRWSTRSSENNEGVIVKVSK
EHVEELTKHAKSVSKKGSEEEGDITNPINLREGEPDLSNNFGKLFEVKPDKKNPQLQDLDM
MLTCVEIKEGALMLPHFNSKAMVIVVVNKGTGNLELVAVRKEQQQRGRREEEEDEDEEEEG
SNREVRRYTARLKEGDVFIMPAAHPVAINASSELHLLGFGINAENNHRIFLAGDKDNVIDQIE
KQAKDLAFPGSGEQVEKLIKNQKESHFVSARPQSQSQSPSSPEKESPEKEDQEEENQGGK
GPLLSILKAFN

SEQ ID NO18: Ara h 3.01.01 as used in example 3

MSHHHHHHHHLEVLFQGPSMRQQPEENACQFQRLNAQRPDNRIESEGGYIETWNPNNQE
FECAGVALSRLVLRRNALRRPFYSNAPQEIFIQQGRGYFGLIFPGCPRHYEEPHTQGRRSQ
SQRPPRRLQGEDQSQQQRDSHQKVHRFDEGDLIAVPTGVAFWLYNDHDTDVVAVSLTDTN
NNDNQLDQFPRRFNLAGNTEQEFLRYQQQSRQSRRRSLPYSPYSPQSQPRQEEREFSPR
GQHSRRERAGQEEENEGGNIFSGFTPEFLEQAFQVDDRQIVQNLRGETESEEEGAIVTVRG
GLRILSPDRKRRADEEEEYDEDEYEYDEEDRRRGRGSRGRGNGIEETICTASAKKNIGRNR
SPDIYNPQAGSLKTANDLNLLILRWLGPSAEYGNLYRNALFVAHYNTNAHSIIYRLRGRAHVQ
VVDSNGNRVYDEELQEGHVLVVPQNFAVAGKSQSENFEYVAFKTDSRPSIANLAGENSVID
NLPEEVVANSYGLQREQARQLKNNNPFKFFVPPSQQSPRAVA

The present invention is further illustrated by the following non-limiting examples from which further features, embodiments, aspects and advantages of the present invention may be taken.

**1. Example 1: Detection of IgE antibodies to Ara h 7 isotypes in sera from peanut-sensitized patients by ELISA**

**1.1. Recombinant proteins**

Heterologous expression and purification of recombinant allergens

Ara h 7.0101 (SEQ ID NO11), Ara h 7.0201(SEQ ID NO12), Ara h 7.0 (SEQ ID NO13), Ara h 6 (SEQ ID NO15) and Ara h 2 (SEQ ID NO1 were expressed and purified as His6 fusion proteins having the sequences indicated by the respective SEQ ID NOs in E. *coli* as described by Sitaru C, Dähnrich C, Probst C, Komorowski L, Blöcker I, Schmidt E, Schlumberger W, Rose C, Stocker W, Zillikens D. Enzyme-linked immunosorbent assay using multimers of the 16th non-collagenous domain of the BP180 antigen for sensitive and specific detection of pemphigoid autoantibodies. Exp Dermatol. 2007 Sep;16(9):770-7. Purification was carried out under denaturing conditions by means of immobilized metal ion chromatography.

**1.2. Detection IgE binding by means of ELISA**

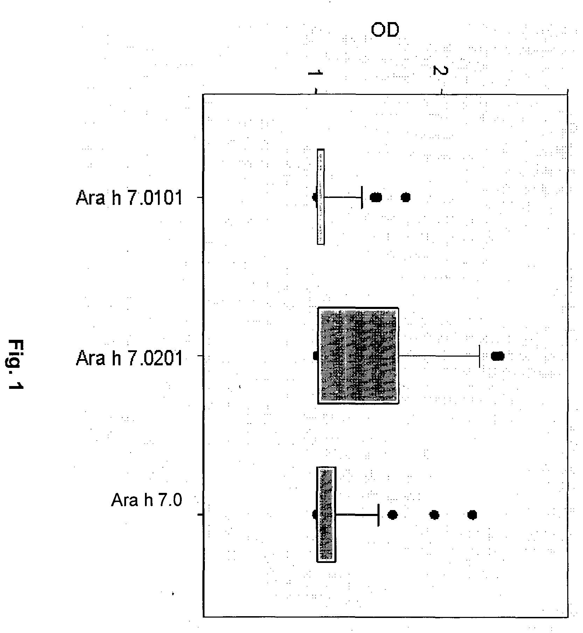

The IgE binding ability of the three Ara h 7 isoforms was analysed by enzyme-linked immunosorbend assay (ELISA) incubated with sera of 33 patients who were IgE positive for peanut extract (**Fig. 1**). In addition 10 sera of atopic patient without peanut sensitization and 23 healthy blood donors were incubated as negative controls (all reacted negative, data not shown).

Microtiter plates were coated with the recombinant peanut allergens (9 $\mu$g/ml in PBS, pH 7.5) 2 h at 4°C as described by Sitaru *et al.* Sera were diluted v/v 1/10 in blocking-buffer (PBS-0,1% bovine serum albumin), 100 $\mu$l per well applied in duplicates to the plates and allowed to react over night at 4°C.

Bound antibodies were detected using anti-human IgE peroxidase conjugate and stained with tetramethylbenzidine (Total IgE ELISA EV 3840-9601 E, Euroimmun, Lubeck, Germany). The optical density (OD) was read at 450 nm with reference at 620 nm using an automated spectrophotometer (Spectra Mini, Tecan, Germany).

**Results**

IgE reactivity against Ara h 7 isotype 7.0101, Ara h 7 isotype 7.0201 and Ara h 7.0 were measured in 66 human Sera. None of the 23 blood donors and none of the 10 sera from allergic patient who had no IgE against peanut extract react positive. Of the 23 peanut extract positive sera 11 showed positive results with Ara h 7.0201, but only 6 and 10 with Ara h 7.0101 and Ara h 7, respectively.

Among the positive reacting sera the average OD was 1.0 for Ara h 7.0201. The average OD for Ara h7.0101 and Ara h 7.0 was quite lower (both 0.4).

These results show, on the one hand, that Ara h 7.0201 reacted stronger with the patient IgE and, on the other hand, that more patients could be correctly diagnosed by detection of IgE antibodies using Ara h7.0201 as target antigen compared to the other two isoforms (Fig. 1). In fact, some patients can only (!) be identified and diagnosed as allergic if antibodies to Ara h 7.0201 are detected.

**Example 2: Detection of IgE antibodies by Line blot**

The IgE binding ability of the 2S albumins Ara h 2, Ara h 6, and three Ara h 7 isoforms were investigated by immunoblot analysis. Serum samples from 34 peanut sensitized subjects (**Fig**. **2**) were incubated with Line blot-immunoblots and intensities were evaluated with the EUROLINEScan software. In addition 20 sera of atopic subjects without peanut sensitization and 17 healthy blood donors were incubated as negative controls (all negative, data not shown).

For the Line blot immunoblots, the recombinant allergens were coated on nylon-based membranes using standard methods. Afterwards the membranes were blocked, dried and fixed on a foil. This foil was cut into strips which were incubated with serum.

Manual incubation of serum samples was carried out according to the EUROIMMUN immunoblot instructions (for Example in DPA-Dx pollen 1, DP 3210-1601-1 E). All incubation and washing steps were done on a rocking shaker at room temperature (+18°C to +25°C). Reagents were taken from a kit supplied by EUROIMMUN (DPA-Dx pollen 1, DP 3210-1601-1 E). The immunoblot strips were preincubated with working strength universal buffer (WSUB) for 5 minutes. After removing all liquid strips were in a first step incubated with 100 $\mu$l of each serum sample diluted with 1.0 ml WSUB overnight (12 to 24 h). In a second step the strips were incubated with 1.0 ml enzyme conjugate (alkaline phosphatase-conjugated antihuman IgE) for 60 minutes. After step one and two the liquid was respectively aspirated off and the strips were washed for 3 x 5 minutes with 1.0 ml WSUB . In the third step the strips were incubated with 1.0 ml chromogen/substrate solution for 10 minutes. Afterwards the liquid was again aspirated off and the enzyme reaction was stopped by washing each strip 3 x 1 minute with deionised or distilled water. Finally the test strips were placed on the evaluation protocol, air dried and evaluated with the EUROLINEScan software.

Results

In total 71 samples were analysed with the immunoblot. In none of the 20 sera from atopic subjects without specific IgE (sIgE) against peanut extract and the 17 healthy blood donors sIgE against Ara h 2, Ara h 6, and all three isoforms of Ara h 7 was detected. Of the 34 samples from subjects with sIgE against peanut extract 17 samples reacted positive with Ara h 2 and Ara h 6, 15 with Ara h 7.0201, 14 with Ara h 7.0101 and 11 with Ara h 7.0, respectively.

While among the positive reacting sera the mean of immunoblot intensities from Ara h 2, Ara h 6 and Ara h 7.0201 was 84, 59, and 74, average intensities from Ara h 7.0101 and Ara h 7 were quite lower (20 and 28) (**Fig**. **2**).

The results show that subjects with sIgE against peanut extract present higher average IgE levels of Ara h 7.0201 than of Ara h 7.0101 and Ara h 7.0. Furthermore peanut extract IgE positive subjects have very similar average levels of Ara h 2 and Ara h 7.0201 IgE indicating that Ara h 7.0201 IgE could be another major allergen component for peanut allergy as well as Ara h 2.

**Example 3: Study based on larger patient cohort using Lineblot and ELISA**

*Patient selection*

To evaluate the diagnostic value of the different peanut storage proteins, adults with a suspected peanut allergy based on history and/or sensitization who had undergone a diagnostic open or double-blind placebo-controlled food challenge (DBPCFC) with peanut in the University Medical Center Utrecht between 2003 and September 2014 were selected (n = 127). Residual serum after routine blood collection one year before or after the food challenge date was available for 95 subjects. From this cohort, 40 peanut allergic and 40 tolerant patients (i.e. with a positive and negative challenge respectively) were selected based on availability of ImmunoCAP ISAC sensitization data. The DBPCFCs were performed in accordance with the international consensus protocol as described before (Taylor SL, Hefle SL, Bindslev-Jensen C, Atkins FM, Andre C, Bruijnzeel-Koomen C, et al. A consensus protocol for the determination of the threshold doses for allergenic foods: How much is too much? Clin Exp Allergy 2004;34:689-95. doi:10.1111/j.1365-2222.2004.1886.x.). All positive challenges were DBPCFCs. Negative challenges also included two open peanut challenges. For the inhibition experiments, serum was gathered from 10 allergic adults sensitized to peanut extract and with a positive DBPCFC, randomly selected from a cohort previously characterized by Peeters KABM, Koppelman SJ, van Hoffen E, van der Tas CWH, den Hartog Jager CF, Penninks AH, et al. Does skin prick test reactivity to purified allergens correlate with clinical severity of peanut allergy? Clin Exp Allergy 2007;37:108-15. doi:10.1111/j.1365-2222.2006.02628.x.

*LINE BLOT*

Sensitization to recombinant peanut storage proteins Ara h 1.0101, 2.0201, 3.0101, 6.0101 and Ara h 7 isotype 7.0201 (**Table 1**) was assessed using a line blot as described in example 2. Briefly, the test strips were incubated at room temperature on an orbital shaker overnight with 100 $\mu$l of patient sera diluted 1:11 in dilution buffer. After washings, an incubation with an enzyme-labelled anti-human IgE antibody, and then with the substrate nitro-blue tetrazolium/5-bromo-4-chloro-3'-indolyphosphate, the reaction was evaluated using the software "EUROLineScan". The intensity of the bands was reported as an intensity level and a class, corresponding to the Enzyme-Allergo-Sorbent Test classification (class 0-6) (Williams PB, Barnes JH, Szeinbach SL, Sullivan TJ. Analytic precision and accuracy of commercial immunoassays for specific IgE: Establishing a standard. J Allergy Clin Immunol 2000;105:1221-30. doi:10.1067/mai.2000.105219). A class of 1 or higher, corresponding to intensity level of 3 or higher, was considered positive.

*Heterologous expression and purification of recombinant allergens*

Ara h 7.0101 (SEQ ID NO11), Ara h 7.0201 (SEQ ID NO12), Ara h 7 (SEQ ID NO13), Ara h 6 (SEQID NO15) and Ara h 2 (SEQ ID NO14) were expressed without signal sequence as fusion proteins with N-terminal His(6x) in *E. coli* with represented by the respective SEQ ID NOs as described by Sitaru *et al.* (2007). Purification was carried out under denaturing conditions by means of immobilized metal ion chromatography. For inhibition experiments recombinant proteins were dialyzed against TBS (20 mM Tris-HCl, pH 7.4, 150 mM NaCl, 3% Sucrose, 1.2 mM Glutathion, non-reduced, 3.8 mM Glutathion, reduced).

*ELISA and inhibition experiment*

Microtiter plates were coated with immobilized recombinant peanut allergens (9 $\mu$g/ml in PBS, pH 7.5) 2 h at 4°C as described by Sitaru *et al...*
For dose-dependent cross-inhibition experiments, optimal diluted sera in blocking buffer described in example 1 (see **Table 3**) were pre-incubated with the competing allergens at final concentration of 0.1, 1 and 10 $\mu$g/ml, for 30 min under shaking at room temperature before applying to the plates. Inhibition values are given as a percentage reduction of OD compared to the controls, in which only blocking buffer has been added.

*Data analysis and statistics*

Spearman correlation was used to analyze the correlation between results of the sIgE tests. The chi-squared test was used to assess differences in positive tests between tolerant and allergic patients. The area under the receiver operating characteristic (ROC) curve (AUC) was determined to evaluate the ability of the tests to discriminate between allergic and tolerant, as established by food challenge. SPSS Statistics 21 (IBM Corporation, Armonk, NY, USA) was used to perform the analyses.

**Results**

*Evaluation of sensitization to peanut storage proteins*

Patient characteristics of the 80 peanut challenged patients, used in the diagnostic evaluation, are listed in **Table 2**. Median age was 25 years (range: 16-77) and 36% was male. Sensitization to all peanut storage proteins occurred significantly more often in the peanut allergic group. In the peanut allergic group, 73 % was sensitized to at least one of the five storage proteins, compared to 15 % in the peanut tolerant group. Peanut 2S albumin Ara h 2 was most recognized in the peanut allergic patients (65%), closely followed by the other two 2S albumins Ara h 6 and 7 (both 60%).
The highest discriminative value was found for Ara h 6 (AUC 0.85), followed by Ara h 7 and 2 (AUC 0.83 and 0.81). EUROLINE intensity values showed a strong to very strong correlation with ISAC ISU results for the peanut storage proteins ($r_s$ value Ara h 1: 0.73, Ara h 2: 0.87, Ara h 3: 0.80, Ara h 6: 0.84, all p<0.001). Ara h 7 intensity values strongly correlated with those from Ara h 2 and 6 on EUROLINE ($r_s$ = 0.81 and p<0.001 for both). Sensitization results as well as discriminative ability for components on both EUROLINE and ImmunoCAP ISAC are listed in **Table 2**.
Co-sensitization to peanut storage proteins was most common. Almost half of all patients sensitized to peanut storage proteins Ara h 1, 2, 3, 6 or 7 was sensitized to all five (46%; Figure E1 in the Online Repository), followed by co-sensitization to Ara h 2, 6 and 7 only (14%). When looking specifically at patients sensitized to 2S albumins Ara h 2, 6 or 7, the majority was co-sensitized to all three (n=24, 68%; Figure 1). Mono-sensitization was observed for Ara h 2 (n=6), Ara h 6 (n=2) and Ara h 7 (n=2), with a positive peanut challenge in 4 out of 6, 1 out of 2 and 1 out of 2 respectively.

*ELISA inhibition with Ara h 2, 6 and 7 isoforms*

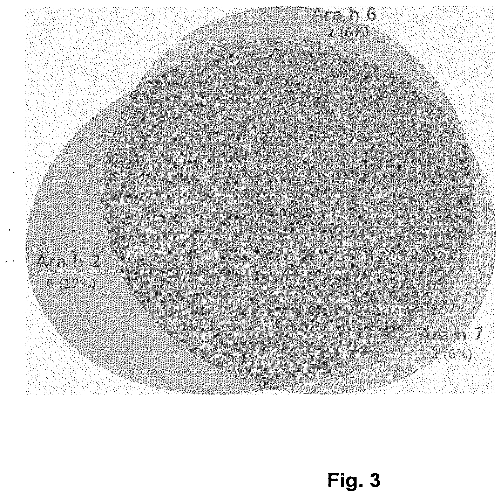

For the 10 patients in the inhibition study, median age was 25 years and four were male. Sensitization to peanut extract was detected in all subjects, with ImmunoCAP titers ranging from 1.7 to >100 kU/L. Firstly, IgE reactivity against the three Ara h 7 isoforms, as well as Ara h 2 and 6 was assessed in the 10 sera by means of ELISA. Four sera showed reactivity to all five components. Additionally, two sera reacted to Ara h 7.0201, Ara h 2 and 6 and one serum to Ara h 2 and 6 only. In the remaining three patients OD values were too low for inhibition experiments. Optimal dilutions were determined for each serum (data not shown). The cross inhibition experiments illustrated that there was variability per patient serum in the maximum amount of inhibition obtained between the different 2S albumins (Fig. 3). In some patients, no inhibition was observed, while others demonstrated partly or almost complete inhibition with the different immobilized allergens and inhibitors. For both the Ara h 7.0101 and 7 isoforms, the other two isoforms were able to achieve strong inhibition, similar to inhibition with the same allergen. For Ara h 7.0201, inhibition with the other two epitopes resulted in very limited inhibition.

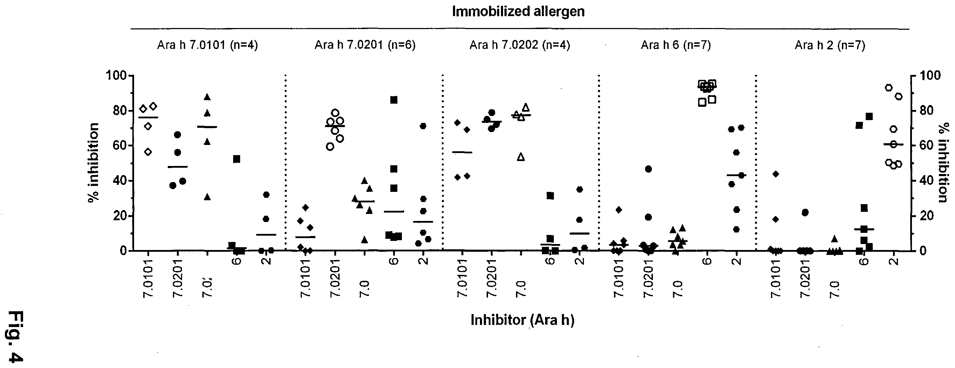

The variability in inhibition was even more pronounced between the different 2S albumins (**Fig. 4**). For example for immobilized Ara h 7.0201, inhibition with Ara h 6 resulted in 8 to 86 % inhibition (median 22 %) and with Ara h 2 inhibition of 4 to 71 % (median 16 %). On the other hand, binding to Ara h 6 and Ara h 2 was inhibited by Ara h 7 isoforms, but inhibition of sIgE against Ara h 6 by Ara h 2 ranged from 12 to 70 % (median 43%) and vice versa up to 77% (median 24 %). In summary, Ara h 2 and 6 were able to inhibit binding to Ara h 7.0201 in some patients, but not others. The Ara h 7 isoforms were poorly able to inhibit binding to Ara h 2 and 6, although Ara h 2 and 6 were able to inhibit binding to each other in a varying degree.

**Discussion**

In our cohort of 80 peanut challenged patients, we demonstrated that Ara h 7.0201 has a discriminative ability very similar to the major allergens Ara h 2 and 6, which can be explained by their frequent co-sensitization, together with a strong correlation between their results. Overall, we found that Ara h 6 had the best discriminative ability, slightly higher than Ara h 2, on both ImmunoCAP ISAC as on the EUROLINE system.

Besides the common co-sensitization, we also observed mono-sensitization to Ara h 2, 6 or 7.0201. While mono-sensitization to Ara h 2 was most-common (n=6), it is important to acknowledge the presence of mono-sensitization to Ara h 6 and 7.0201, which would be missed when only testing for sensitization to Ara h 2. This is the first study demonstrating mono-sensitization to Ara h 7.0201 in two subjects.

Previous studies have investigated sensitization to Ara h 7, but only the Ara h 7 01.01 isoform. After first cloning Ara h 7.0101, Kleber-Janke et al. detected sensitization to rAra h 7.0101 in 17 out of 40 (43%) peanut sensitized subjects with a convincing history of peanut allergy, compared to 85% for Ara h 2. Codreanu and colleagues investigated the role of several recombinant peanut in an immunoassay and demonstrated that rAra h 7.0101 has a poor sensitivity compared to rAra h 2 and 6.

The inhibition experiments in our study illustrated that Ara h 7.0201 is the most relevant isoform of Ara h 7. In sera of patients with sIgE against Ara h 7.0101 and 7.0, strong inhibition by the other isoforms suggest recognition of epitopes present on all three Ara h 7 isoforms. On the other hand, in subjects sensitized to Ara h 7.0201 a lack of inhibition by the other isoforms indicate co-sensitization to unique epitopes, not present on the other two isoforms. In four of the six sera sensitized to any Ara h 7 isoform, there was co-sensitization to all three Ara h 7 isoforms. It appears contradicting that binding to Ara h 7.0201 could not be inhibited by the other isoforms, while the other way around Ara h 7.0201 was able to inhibit binding to the other isoforms. One explanation is recognition of multiple epitopes of Ara h 7.0201, both unique and cross-reactive, in a single patient, where abundant sIgE against the unique epitope(s) present on one Ara h 7 isoform result in low inhibition with other isoforms lacking that epitope. Another influencing factor could be the difference in accessibility of epitopes for IgE between the immobilized coated allergens on the solid phase versus the dissolved, folded allergens.

Cross-inhibition of 2S albumins showed that IgE against Ara h 2 and 6 could not be inhibited by Ara h 7 isoforms, but to some extend by each other. This could be explained by shared epitopes on Ara h 2 and 6 that are not part of Ara h 7 isoforms.

In conclusion, this study has demonstrated that Ara h 7 isotype 7.0201 is the third clinically relevant peanut 2S albumin, with on population level a discriminative ability for peanut allergy comparable to Ara h 2 and 6. While co-sensitization to peanut storage proteins, and more specifically 2S albumins, is most common, mono-sensitization to either Ara h 2, 6 or 7 occurs in individual patients, leading to a risk of misdiagnosis when testing for a single 2S albumin.

**Example 4: Detection of IgE antibodies against specific Ara h 7 epitopes using peptide microarrays**

**Peptide microarray incubation and visualization**

A peptide microarray comprising 15mer peptides of Ara h 7.0201 (SEQ ID NO2) with overlapping sequences (offset: 1, every peptide printed in dublicates) was commercially obtained (PEPperPRINT), covering the C-terminus of Ara h 7.0201 (SEQ ID NO6). For the incubation experiments the microarray was blocked with working strength universal buffer (WSUB, see example 2) for 1 h at RT on an orbital shaker. All further described incubations were also carried out in WSUB. Sera of 3 peanut positive and 2 peanut negative patients were diluted 1:4 and incubated overnight at 4 °C. For the detection of bound IgE antibodies, biotinylated anti-IgE IT-28 (Squarix) was diluted 1:5000 and incubated on the array for 1 h at room temperature. After washing, the array was incubated for 1 h at room temperature with fluorescent Neutravidin 800 (Thermo Fisher), diluted 1:5000. The peptide microarray slides were scanned with a Licor Odyssey Imager at a wavelength of 800 nm (intensity: 8.5). Image focus was set to 0.8 mm and the maximum image resolution (21 $\mu$m) was chosen to guarantee maximum sensitivity.

**Evaluation**

After scanning, TIFF images and peptide map files were loaded to the Pepslide Analyzer Software (SICASYS) for quantitation of the signals corresponding to every single peptide. The raw data was exported as CSV files and the log2 of the signal-to-noise ratio for each single Ara h 7 peptide ($S_{Ara}$) and for empty spots ($S_{Blank}$) on the array was calculated. For a more robust and normalized evaluation, z-scores for every single peptide (Zi) were calculated according to the following formula:

$$Z_i = \frac{S_i - Median\,(S_{Blank})}{MAD\,(S_{Blank})}$$

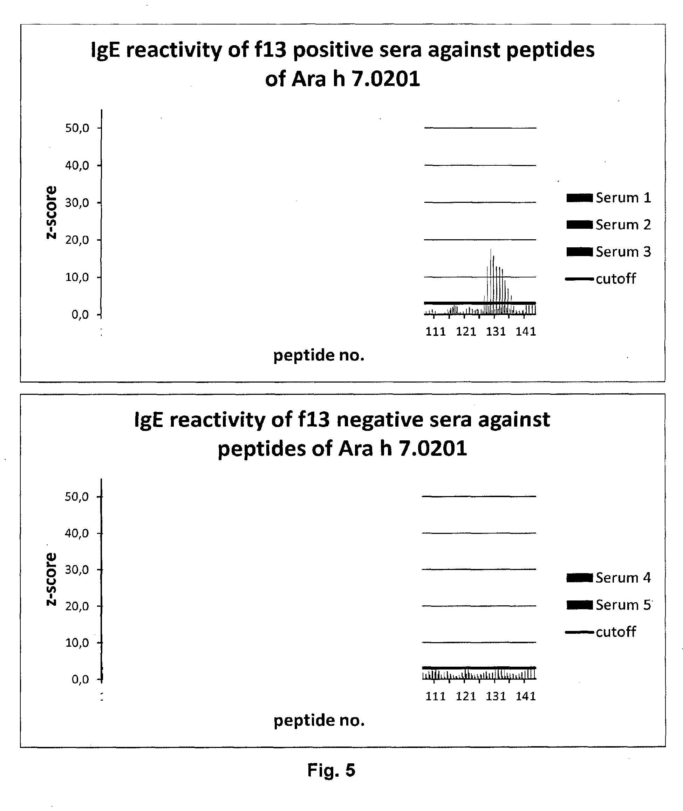

Based on those calculations, a positive binding epitope was defined as the detection of at least 6 subsequent peptides comprising a z-score of 3.0 or higher (p=0.003). Data visualization was performed using Microsoft Excel (Fig.5).

**Results**

While no epitopes were detected by the two negative sera, using serum 3, a previously unknown Ara h 7.0201 epitope could be detected comprising the sequence GFRSPS (amino acids 129-134) (amino acid residue numbers according to SEQ ID NO2). Although below the z-score cutoff, this epitope is also detectable for serum 1. Since this sequence is unique to Ara h 7.0201, it can be concluded that this epitope is the one associated with the enhanced reactivity of Ara h 7.0201 compared to the two other isoforms. Taken together, our results reveal for the first time two binding epitopes for Ara h 7.

**Tables**

Table 1: Peanut storage proteins present on the EUROLINE strip. Adapted from Becker and Jappe [5] and Van Erp et al [7].

| Allergen | Protein superfamily | Proportion of total protein | Sedimentation coefficient | (Assumed) biological function | Aliases |
|---|---|---|---|---|---|
| **Ara h 1** | Cupin superfamily | 11-31% | 7S vicilin | Storage protein | Conarachin |
| **Ara h 2** | Prolamin superfamily | 7-16% | 2S albumin | Storage protein, trypsin inhibitor | Conglutin |
| **Ara h 3** | Cupin superfamily | 38-76% | 11S legumin, glycinin | Storage protein | Arachin |
| **Ara h 6** | Prolamin superfamily | 4-14% | 2S albumin | Storage protein | Conglutin |

(continued)

| Allergen | Protein superfamily | Proportion of total protein | Sedimentation coefficient | (Assumed) biological function | Aliases |
|---|---|---|---|---|---|
| Ara h 7 | Prolamin superfamily | 0.5% [16] | 2S albumin | Trypsin/amylase inhibitor [4], storage protein | Conglutin |

**Table 2**: Patient characteristics and sensitization data of the 40 peanut tolerant and 40 peanut allergic patients in the cohort

| Characteristic / sensitization | Overall (n=80) | | Peanut tolerant (n=40) | | Peanut allergic (n=40) | | p value† | Tolerant versus allergic | |
|---|---|---|---|---|---|---|---|---|---|
| | | | | | | | | AUC | (95%CI) |
| Age (median [IQR]) | 25 | (21-37) | 31 | (22-43) | 23 | (20-29) | 0.01 | - | - |
| Sex (n male [%]) | 29 | (36%) | 12 | (30%) | 17 | (43%) | 0.25 | - | - |
| EUROLINE* | | | | | | | | | |
| - Ara h 1 | 20 | (25%) | 3 | (8%) | 17 | (43%) | <0,001 | 0,69 | (0,57-0,81) |
| -Arah 2 | 30 | (38%) | 4 | (10%) | 26 | (65%) | <0,001 | 0,81 | (0,71-0,91) |
| -Arah 3 | 17 | (21%) | 2 | (5%) | 15 | (38%) | <0,001 | 0,72 | (0,61-0,84) |
| -Arah 6 | 27 | (34%) | 3 | (8%) | 24 | (60%) | <0,001 | 0,85 | (0,76-0,93) |
| -Arah 7 | 27 | (34%) | 3 | (8%) | 24 | (60%) | <0,001 | 0,83 | (0,73-0,92) |

IQR: Interquartile range. AUC: Area under the curve. CI: confidence interval
* Using manufacturer's recommended cutoff values for a positive test. † p value of difference in number of positive tests between tolerant and allergic subjects (chi-squared test). ‡ n = 79

Table 3: Individuell Dilution of the sera for dose dependend inhibition experiment described in example 3

| | ELISA coated with | | | | |
|---|---|---|---|---|---|
| Serum No | Ara h 7.0101 | mAra h 7.0201 | Ara h 7.0 | Ara h 6.0101 | -Ara h 2.0201 |
| 1 | 1:5 | 1:5 | 1:5 | 1:5 | 1:5 |
| 2 | 1:5 | 1:10 | 1:5 | 1:20 | 1:10 |
| 3 | 1:5 | 1:10 | 1:5 | 1:10 | 1:5 |
| 4 | 1:5 | 1:5 | 1:5 | 1:5 | 1:5 |
| 5 | ----- | 1:5 | ----- | 1:5 | 1:5 |
| 6 | ----- | ----- | ----- | 1:5 | 1:5 |
| 7 | ----- | 1:5 | ----- | 1:5 | 1:5 |

**Claims**

1. A diagnostically useful carrier comprising a means for specifically capturing an antibody to Ara h 7 isotype 7.0201, preferably SEQ ID NO 6, more preferably to a sequence from the group comprising SEQ ID NO7, SEQ ID NO8, SEQ ID NO9 and SEQ ID NO10, most preferably to SEQ ID NO8, in a sample from a subject, wherein the carrier is preferably selected from the group comprising a bead, a test strip, a microtiter plate, a blot, preferably from the group comprising western blot, line blot and dot blot, a glass surface, a slide, a biochip and a membrane, and is most preferably a microtiter plate or a line blot.

2. The diagnostically useful carrier according to claim 1, wherein the carrier further comprises a means for specifically capturing an antibody to one or more further antigens from the group comprising Ara h 2, Ara h 6, Ara h 1, Ara h 3, Ara h 9, Ara h 8 and Ara h 5, more preferably from the group comprising Ara h 2, Ara h 6, Ara h 1, Ara h 3, Ara h 9, more preferably an antibody to Ara h 2 and/or an antibody to Ara h 6, most preferably an antibody to Ara h 2

3. A kit comprising the diagnostically useful carrier according to any of claims 1 to 2, optionally as well as a means for specifically detecting a captured antibody.

4. The kit according to claim 3,
   wherein the diagnostically useful carrier comprises a means for specifically capturing an antibody to one or more further antigens from the group Ara h 2, Ara h 6, Ara h 1, Ara h 3, Ara h 9, Ara h 8 and Ara h 5,
   wherein the means for specifically capturing an antibody to isotype 7.0201, preferably SEQ ID NO 6, more preferably to a sequence from the group comprising SEQ ID NO7, SEQ ID NO8, SEQ ID NO9 and SEQ ID NO10, most preferably SEQ ID NO8, and the means for specifically capturing an antibody to one or more further antigens are on separate carriers
   or
   wherein the means for specifically capturing an antibody to Ara h 7 isotype 7.0201, preferably SEQ ID NO 6, more preferably to a sequence from the group comprising SEQ ID NO7, SEQ ID NO8, SEQ ID NO9 and SEQ ID NO10, most preferably SEQ ID NO8, and the means for specifically capturing an antibody to one or more further antigens are on one, preferably covalently linked to one carrier.

5. A method comprising the step detecting in a sample from a subject the presence of an antibody to Ara h 7 isotype 7.0201, preferably to SEQ ID NO 6, more preferably to a sequence from the group comprising SEQ ID NO7, SEQ ID NO8, SEQ ID NO9 and SEQ ID NO10, most preferably SEQ ID NO8.

6. The method according to claim 5, further comprising the step detecting in a sample from a subject the presence of an antibody to one or more further antigens; preferably selected from the group comprising Ara h 2, Ara h 6, Ara h 1, Ara h 3, Ara h 9, Ara h 8 and Ara h 5, more preferably from the group comprising Ara h 2, Ara h 6, Ara h 1, Ara h 3 and Ara h 9, most preferably Ara h 2 and/or Ara h 6.

7. The method according to any of claims 5 to 6, wherein the presence or absence of an antibody to Ara h 7 isotype 7.0201 and the presence of an antibody to one or more further antigens, preferably Ara h 2 and/or Ara h 6, is detected simultaneously.

8. The method according to any of claims 5 to 7, wherein the presence or absence of an antibody to Ara h 7 isotype 7.0201, preferably SEQ ID NO8 and the presence of an antibody to one or more further antigens is detected in spatially separate binding reactions.

9. The method according to any of claims 5 to 8, wherein the presence of an antibody Ara h 7 isotype 7.0201 and the presence of an antibody to one or more further antigens is detected in a one-pot reaction.

10. The carrier, kit or method according to any of claims 1 to 9, wherein the antibody to isotype 7.0201 is an antibody monospecific to Ara h 7, preferably to isotype 7.0201, preferably SEQ ID NO 6, more preferably a sequence from the group comprising SEQ ID NO7, SEQ ID NO8, SEQ ID NO9 and SEQ ID NO10, most preferably SEQ ID NO8.

11. A pharmaceutical composition comprising a polypeptide comprising SEQ ID NO 8, more preferably a sequence from the group comprising SEQ ID NO6, SEQ ID NO7, SEQ ID NO8, SEQ ID NO9 and SEQ ID NO10, more preferably SEQ ID NO6, most preferably Ara h 7 isotype 7.0201 or a variant thereof and preferably one or more further antigens from the group comprising Ara h 2, Ara h 6, Ara h 1, Ara h 3, Ara h 9, Ara h 8 and Ara h 5 and a variant thereof, more preferably from the group comprising Ara h 2, Ara h 6, Ara h 1, Ara h 3, Ara h 9 and a variant thereof, most preferably Ara h 2 and/or Ara h 6.

12. A polypeptide comprising SEQ ID NO 8, more preferably a sequence from the group comprising SEQ ID NO6, SEQ ID NO7, SEQ ID NO8, SEQ ID NO9 and SEQ ID NO10, more preferably SEQ ID NO6, most preferably Ara h 7 isotype 7.0201 or a variant thereof, wherein the polypeptide is recombinant, immobilized purified or a fusion protein, preferably purified and recombinant and immobilized.

13. A use of the polypeptide according to claim 12, the carrier according to any of claims 1 to 2 or the kit according to any of claims 3 to 4 for the diagnosis of peanut allergy, wherein preferably the sensitivity of the diagnosis is increased.

14. A use of the polypeptide according to claim 12 for the manufacture of a kit for the diagnosis of peanut allergy, wherein preferably the sensitivity of the diagnosis is increased.

15. The pharmaceutical composition according to claim 11, the polypeptide according to claim 12 or the use according to any of claims 13 to 14, wherein the polypeptide comprising SEQ ID NO 8, more preferably a sequence from the group comprising SEQ ID NO6, SEQ ID NO7, SEQ ID NO8, SEQ ID NO9 and SEQ ID NO10, more preferably SEQ ID NO6, most preferably Ara h 7 isotype 7.0201 or a variant thereof is a composition comprising said polypeptide and Ara h 2 and/or Ara h 6 or a variant thereof.

16. An antibody, preferably IgE class antibody, binding specifically to Ara h 7 isotype 7.0201, preferably SEQ ID NO6, more preferably a sequence from the group comprising SEQ ID NO7, SEQ ID NO8, SEQ ID NO9 and SEQ ID NO10, most preferably to SEQ ID NO8, which antibody is preferably isolated.

Fig. 1

**Fig. 2**

**Fig. 3**

Fig. 4

## IgE reactivity of f13 positive sera against peptides of Ara h 7.0201

z-score

50,0
40,0
30,0
20,0
10,0
0,0

111 121 131 141

peptide no.

▬ Serum 1
▬ Serum 2
▬ Serum 3
— cutoff

## IgE reactivity of f13 negative sera against peptides of Ara h 7.0201

z-score

50,0
40,0
30,0
20,0
10,0
0,0

111 121 131 141

peptide no.

▬ Serum 4
▬ Serum 5
— cutoff

**Fig. 5**

Europäisches
Patentamt

European
Patent Office

Office européen
des brevets

# EUROPEAN SEARCH REPORT

**Application Number**

EP 17 00 0245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/172511 A1 (SANOFI SA [FR]) 27 October 2016 (2016-10-27) | 11,12,15 | INV. G01N33/68 |
| Y | * abstract * * page 15, paragraph 2 - page 17, last paragraph * * claims 1,14,15 * | 1-10,13, 14 | A61K39/35 C07K14/415 |
| X | WO 2016/131000 A2 (VIRTICI, LLC [US]) 18 August 2016 (2016-08-18) | 11,12 | |
| Y | * abstract * * page 13 * * claims 1-6,22 * | 1-10,13, 14 | |
| X,D | SCHMIDT H. ET AL.: "Detection and structural characterization of natural Ara h 7, the third peanut allergen of the 2S albumin family", J. PROTEOME RES., vol. 9, no. 7, 2 July 2010 (2010-07-02), pages 3701-3709, XP55237195, | 12,16 | |
| Y | * abstract * * figure 2 * * page 3705, column 2, paragraph 2 * * page 3707, column 2, last paragraph - page 3708, column 1, paragraph 1 * | 1-10,13, 14 | **TECHNICAL FIELDS SEARCHED (IPC)** G01N A61K C07K |
| Y | WO 2014/182932 A1 (THE BOARD OF TRUSTEES OF THE LELAND STANFORD JUNIOR UNIVERSITY [US]) 13 November 2014 (2014-11-13) * abstract * * paragraphs [0047], [0052], [0056] * * claims 1-31 * | 1-10,13, 14 | |
| A | WO 2012/129246 A2 (THE REGENTS OF THE UNIVERSITY OF COLORADO, A BODY CORPORATE [US]) 27 September 2012 (2012-09-27) * the whole document * | 1-16 | |

The present search report has been drawn up for all claims

| Place of search | Date of completion of the search | Examiner |
|---|---|---|
| Munich | 8 June 2017 | Giry, Murielle |

## ANNEX TO THE EUROPEAN SEARCH REPORT
## ON EUROPEAN PATENT APPLICATION NO.

EP 17 00 0245

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2017

| Patent document cited in search report | | | Publication date | Patent family member(s) | | | Publication date |
|---|---|---|---|---|---|---|---|
| WO 2016172511 | A1 | | 27-10-2016 | TW<br>US<br>WO | 201709926<br>2016310589<br>2016172511 | A<br>A1<br>A1 | 16-03-2017<br>27-10-2016<br>27-10-2016 |
| WO 2016131000 | A2 | | 18-08-2016 | NONE | | | |
| WO 2014182932 | A1 | | 13-11-2014 | US<br>WO | 2016058377<br>2014182932 | A1<br>A1 | 03-03-2016<br>13-11-2014 |
| WO 2012129246 | A2 | | 27-09-2012 | EP<br>US<br>WO | 2694966<br>2014080730<br>2012129246 | A2<br>A1<br>A2 | 12-02-2014<br>20-03-2014<br>27-09-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013041540 A

**Non-patent literature cited in the description**

- **FLINTERMAN, A. E. ; VAN HOFFEN, E. ; DEN HARTOG, JAGER C. F. ; KOPPELMAN, S. ; PASMANS, S. G. ; HOEKSTRA, M. O. ; BRUIJNZEEL-KOOMEN, C. A. ; KNULST, A. C. ; KNOL, E. F.** Children with peanut allergy recognize predominantly Ara h 2 and Ara h 6, which remains stable over time. *Clin Exp Allergy 2007,* 2007, vol. 37, 1221-1228
- **DE LEON, M. P. ; DREW, A. C. ; GLASPOLE, I. N. ; SUPHIOGLU, C. ; O'HEHIR, R. E. ; ROLLAND, J. M.** IgE cross-reactivity between the major peanut allergen Ara h 2 and tree nut allergens. *Mol Immunol 2007,* 2007, vol. 44, 463-471
- **SCHMIDT, H. ; KRAUSE, S. ; GELHAUS, C. ; PETERSEN, A. ; JANSSEN, O. ; BECKER, W. M.** Detection and structural characterization of natural Ara h 7, the third peanut allergen of the 2S albumin family. *J Proteome Res 2010,* 2010, vol. 9, 3701-709
- **SCHMIDT et al.** *Detection and Structural Characterization of Natural Ara h 7, the Third Peanut Allgergen of the 2S Albumin Family,* 2008
- **PEETERS, K. A. ; KOPPELMAN, S. J. ; VAN HOFFEN, E. ; VAN DER TAS, C. W. ; DEN HARTOG, JAGER C. F. ; PENNINKS, A. H. ; HEFLE, S. L. ; BRUIJNZEEL-KOOMEN, C. A. ; KNOL, E. F. ; KNULST, A. C.** Does skin prick test reactivity to purified allergens correlate with clinical severity of peanut allergy?. *Clin Exp Allergy,* January 2007, vol. 37 (1), 108-15
- **CODREANU, F. ; COLLIGNON, O. ; ROITEL, O. ; THOUVENOT, B. ; SAUVAGE, C. ; VILAIN, A. C. ; COUSIN, M. O. ; DECOSTER, A. ; RENAUDIN, JM. ; ASTIER, C.** *Int Arch Allergy Immunol, 2011,* 2011, vol. 154 (3), 216-26
- **RAOULT, D. ; DASCH, G. A.** The line blot: an immunoassay for monoclonal and other antibodies. Its application to the serotyping of gram-negative bacteria. *J. Immunol. Methods,* 1989, vol. 125 (1-2), 57-65
- **ARTHUR LESK.** Introduction to bioinformatics. Oxford University Press, 2008

- **LARKIN, M. A. ; BLACKSHIELDS, G. ; BROWN, N. P. ; CHENNA, R. ; MCGETTIGAN, P. A. ; MCWILLIAM, H. ; VALENTIN, F. ; WALLACE, I. M. ; WILM, A. ; LOPEZ, R.** Clustal W and Clustal X version 2.0. *Bioinformatics,* 2007, vol. 23, 2947-2948
- **SAMBROOK, J. ; FRITSCH, E. F. ; MANIATIS, T.** Molecular Cloning. CSH, 1989
- **BROWN T. A.** Gene Cloning - an introduction. Chapman & Hall, 1986
- Strategies for Protein Purification. Antibody Purification. GE Healthcare Life Sciences
- **BURGESS, R. R. ; DEUTSCHER, M. P.** Guide to Protein Purification. 2009
- **ZANE, H. D.** Immunology - Theoretical & Practical Concepts in Laboratory Medicine. W. B. Saunders Company, 2001
- **HAUSMANN, O. V. ; GENTINETTA, T. ; BRIDTS, C. H. ; EBO, E. G.** The Basophil Activation Test in Immediate-Type Drug Allergy. *Immunol. Allergy Clin. N. Am.,* 2009, vol. 29, 555-566
- **SITARU C ; DÄHNRICH C ; PROBST C ; KOMOROWSKI L ; BLÖCKER I ; SCHMIDT E ; SCHLUMBERGER W ; ROSE C ; STOCKER W ; ZILLIKENS D.** Enzyme-linked immunosorbent assay using multimers of the 16th non-collagenous domain of the BP180 antigen for sensitive and specific detection of pemphigoid autoantibodies. *Exp Dermatol.,* September 2007, vol. 16 (9), 770-7
- **TAYLOR SL ; HEFLE SL ; BINDSLEV-JENSEN C ; ATKINS FM ; ANDRE C ; BRUIJNZEEL-KOOMEN C et al.** A consensus protocol for the determination of the threshold doses for allergenic foods: How much is too much?. *Clin Exp Allergy,* 2004, vol. 34, 689-95
- **PEETERS KABM ; KOPPELMAN SJ ; VAN HOFFEN E ; VAN DER TAS CWH ; DEN HARTOG JAGER CF ; PENNINKS AH et al.** Does skin prick test reactivity to purified allergens correlate with clinical severity of peanut allergy?. *Clin Exp Allergy,* 2007, vol. 37, 108-15

• **WILLIAMS PB ; BARNES JH ; SZEINBACH SL ; SULLIVAN TJ.** Analytic precision and accuracy of commercial immunoassays for specific IgE: Establishing a standard. *J Allergy Clin Immunol,* 2000, vol. 105, 1221-30